(19) Europäisches Patentamt
European Patent Office
Office européen des brevets

(11) Veröffentlichungsnummer: **0 243 918**
**A2**

# (12) EUROPÄISCHE PATENTANMELDUNG

(21) Anmeldenummer: **87106068.7**

(22) Anmeldetag: **25.04.87**

(51) Int. Cl.³: **C 07 D 233/90**
C 07 D 233/64, C 07 D 413/04
C 07 D 403/04, C 07 D 417/04
C 07 D 401/04, C 07 F 7/18
C 07 D 401/12, C 07 D 403/12
C 07 D 405/12, A 01 N 43/50

(30) Priorität: **28.04.86 DE 3614364**

(43) Veröffentlichungstag der Anmeldung:
**04.11.87 Patentblatt 87/45**

(84) Benannte Vertragsstaaten:
**AT BE CH DE ES FR GB GR IT LI NL SE**

(71) Anmelder: **HOECHST AKTIENGESELLSCHAFT**
**Postfach 80 03 20**
**D-6230 Frankfurt am Main 80(DE)**

(72) Erfinder: **Schmierer, Roland, Dr.**
**An der Weinleite 5a**
**D-8901 Todtenweis(DE)**

(72) Erfinder: **Mildenberger, Hilmar, Dr.**
**Fasanenstrasse 24**
**D-6233 Kelkheim (Taunus)(DE)**

(72) Erfinder: **Bürstell, Helmut, Dr.**
**Am Hohlacker 65**
**D-6000 Frankfurt am Main(DE)**

## (54) 1-Phenyl-imidazolverbindungen, Verfahren zu ihrer Herstellung und ihre Verwendung als Wachstumsregulatoren.

(57) Die neuen Verbindungen der Formel I

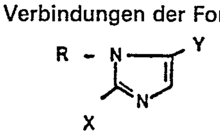

I

worin

R = substituiertes Phenyl

X = einen Rest der Formeln –SH,

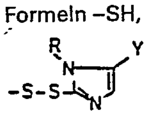

, $-S(O)_m-R^3$, oder Wasserstoff

Y = einen Rest der Formeln

$-CN$, $-\overset{O}{\overset{\|}{C}}-OR^4$, $-\overset{O}{\overset{\|}{C}}-SR^5$, $-\overset{S}{\overset{\|}{C}}-OR^5$,

$-\overset{S}{\overset{\|}{C}}-SR^5$, $-\overset{O}{\overset{\|}{C}}-NR^6R^7$, $-\overset{S}{\overset{\|}{C}}-N(R^6)_2$, $-\overset{NOR^6}{\overset{\|}{C}}-NH_2$,

$-\overset{O}{\overset{\|}{C}}-R^6$, $-\overset{NOR^8}{\overset{\|}{C}}-R^6$, $-CH_2-OR^8$, $-CF_3$,

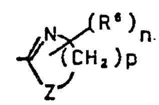
,
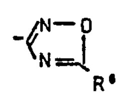
,
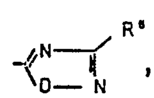
,

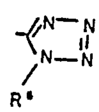
,
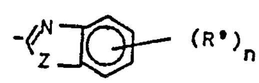

sowie die von $-\overset{O}{\overset{\|}{C}}-R^6$ abgeleiteten Bisulfitaddukte, Acetale, Ketale, Thioacetale oder Thioketale, Z = O, S oder $N-R^6$; m = 0, 1 oder 2; n = 0, 1, 2, 3 oder 4; p = 2 oder 3 bedeuten, sowie deren für landwirtschaftliche Zwecke verträglichen Salze und Quaternisierungsprodukte besitzen vorteilhafte pflanzenwachstumsregulierende Wirkungen. Es werden auch Verfahren zu ihrer Herstellung beschrieben.

EP 0 243 918 A2

Beschreibung:

1-Phenyl-imidazolverbindungen, Verfahren zu ihrer Herstellung und ihre Verwendung als Wachstumsregulatoren

Es ist bekannt, daß 1-Phenyl-imidazol-5-carbonsäurederivate, s. DE-A 32 17 094, pflanzenwachstumsregulierende Eigenschaften besitzen. Überraschenderweise wurde nun gefunden, daß bei Auswahl bestimmter Substituenten am Phenylring die nachfolgenden Imidazolverbindungen in verschiedenen Kulturen vorteilhafte pflanzenwachstumsregulierende Wirkungen besitzen.

Die vorliegende Erfindung betrifft die neuen Verbindungen der Formel I

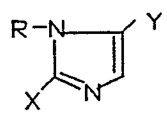

I

worin

R = einen Rest der Formel

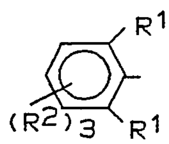

X = einen Rest der Formeln -SH,

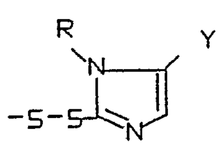

, $-S(O)_m-R^3$, oder Wasserstoff

Y = einen Rest der Formeln

$-CN$, $-\overset{O}{\overset{\|}{C}}-OR^4$, $-\overset{O}{\overset{\|}{C}}-SR^5$, $-\overset{S}{\overset{\|}{C}}-OR^5$, $-\overset{S}{\overset{\|}{C}}-SR^5$, $-\overset{O}{\overset{\|}{C}}-NR^6R^7$,

$-\overset{S}{\overset{\|}{C}}-N(R^6)_2$, $-\overset{NOR^6}{\overset{\|}{C}}-NH_2$, $-\overset{O}{\overset{\|}{C}}-R^6$, $-\overset{NOR^8}{\overset{\|}{C}}-R^6$, $-CH_2-OR^8$, $-CF_3$,

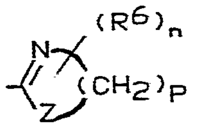 , 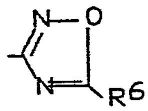 , 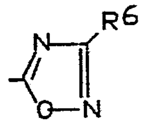 ,

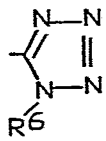 , 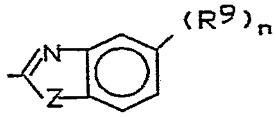

sowie die von $-\overset{\overset{O}{\|}}{C}-R^6$ abgeleiteten Bisulfitaddukte, Acetale, Ketale, Thioacetale oder Thioketale,

Z = O, S oder $N-R^6$

m = 0, 1 oder 2

n = 0, 1, 2, 3 oder 4

p = 2 oder 3

$R^1$= unabhängig voneinander ($C_1$-$C_4$)-Alkyl, das halogeniert sein kann, ($C_2$-$C_3$)-Alkenyl, ($C_2$-$C_3$)-Haloalkenyl, Acetyl, ($C_1$-$C_3$)-Hydroxyalkyl, ($C_1$-$C_4$)-Alkoxy oder Halogen,

$R^2$= unabhängig voneinander Wasserstoff, ($C_1$-$C_4$)-Alkyl, ($C_1$-$C_4$)-Alkoxy oder Halogen,

$R^3$= ($C_1$-$C_4$)-Alkyl, ($C_2$-$C_4$)-Alkenyl, ($C_1$-$C_4$)-Alkoxycarbonyl-($C_1$-$C_2$)alkyl, Phenyl oder Benzyl,

$R^4$= Wasserstoff, ($C_1$-$C_{12}$)-Alkyl, ($C_1$-$C_{12}$)-Alkyl, das ein- bis dreifach durch Hydroxy, Halogen, ($C_1$-$C_6$)-Alkoxy, ($C_1$-$C_4$)-Alkoxy-($C_1$-$C_4$)alkoxy, ($C_1$-$C_4$)-Alkylthio, ($C_1$-$C_4$)-Alkylsulfinyl, ($C_1$-$C_4$)-Alkylsulfonyl, Mono- oder Di-($C_1$-$C_4$-alkyl)amino, Cyano, Aminocarbonyl, ($C_1$-$C_4$)-Alkanoyl, ($C_1$-$C_4$-Alkoxy)carbonyl, Cyclo-($C_3$-$C_7$)alkyl, Tri($C_1$-$C_4$-alkyl)silyl, Benzyloxy,

Benzyloxyethoxy, Phenyl oder Phenyl, das durch Halogen oder $(C_1-C_4)$-Alkyl ein- oder mehrfach substituiert ist, durch Phenoxy, Phenylthio, die beide durch Halogen oder $(C_1-C_4)$-Alkyl ein- oder mehrfach substituiert sein können, durch Oxiranyl, Tetrahydrofuryl, Triazolyl, Pyridinyl, Imidazolyl, Carboxy, Carboxylat mit einem für die Landwirtschaft einsetzbaren Kation oder durch den Rest $-O-N=C(CH_3)_2$ substituiert ist,
$(C_3-C_6)$-Alkenyl, halogeniertes $(C_3-C_6)$-Alkenyl, unsubstituiertes oder durch Halogen oder $(C_1-C_4)$-Alkyl substituiertes Cyclo-$(C_3-C_7)$alkyl, unsubstituiertes oder durch Halogen oder $(C_1-C_4)$-Alkyl substituiertes Cyclo-$(C_5-C_7)$alkenyl, $(C_3-C_6)$-Alkinyl, 1,2-Epoxy-prop-3-yl,
Phenyl oder Phenyl, das ein- oder zweifach durch Halogen, Nitro, Cyano, $(C_1-C_4)$-Alkyl, $(C_1-C_4$-Alkoxy$)$-carbonyl oder $(C_1-C_4)$-Alkoxy substituiert ist,
$(C_1-C_4$-Alkyl$)$carbonyl, Phenylcarbonyl, wobei der Phenylring durch Halogen, Nitro, Cyano oder $(C_1-C_4)$-Alkyl substituiert sein kann,
einen Rest der Formeln $-N=C(R^{10})_2$, $-NR^6R^{11}$,

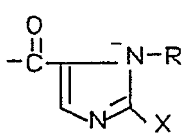 , 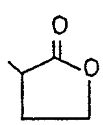 , 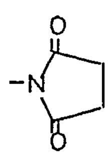 , 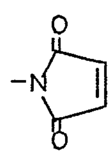 , 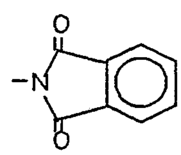

oder ein für die Landwirtschaft einsetzbares Kation,

$R^5$= H, $(C_1-C_{12})$-Alkyl oder $(C_1-C_{12})$-Alkyl, das bis zu zweifach durch $(C_1-C_4)$-Alkoxy-ethoxy, Cyclo-$(C_3-C_6)$-alkyl, Benzyloxy, Phenyl, Phenoxy, $(C_1-C_4)$-Alkylthio, $(C_1-C_4$-Alkoxy$)$carbonyl, Carboxy oder Carboxylat mit einem für die Landwirtschaft einsetzbaren Kation, substituiert ist, Phenyl oder im Falle $-CS-OR^5$ ein für die Landwirtschaft einsetzbares Kation,

$R^6$= jeweils unabhängig voneinander Wasserstoff, $(C_1-C_6)$-Alkyl, Phenyl, Benzyl oder Methylphenyl,

$R^7$= Wasserstoff, ($C_1$-$C_{12}$)-Alkyl oder ($C_1$-$C_{12}$)-Alkyl, das bis zu zweifach, durch ($C_1$-$C_6$)-Alkoxy, ($C_1$-$C_4$)-Alkoxy-ethoxy, Hydroxy, Halogen, Cyclo-($C_3$-$C_6$)alkyl, Benzyloxy, Cyano, Aminocarbonyl, Carboxy, ($C_1$-$C_4$-Alkoxy)carbonyl, Formyl, Phenyl oder Phenoxy substituiert ist,
Phenyl oder Phenyl, das bis zu zweifach durch Halogen, Nitro, Cyano, ($C_1$-$C_4$)-Alkyl oder ($C_1$-$C_4$)-Alkoxy substituiert ist,
($C_3$-$C_6$)-Alkenyl, ($C_3$-$C_6$)-Cycloalkyl, einen Rest der Formeln $-NR^6R^{12}$, $-OR^6$, $-NH-CONH_2$, $-NH-CS-NH_2$ oder $-SO_2R^6$ oder

$R^6$ und $R^7$ gemeinsam mit dem Stickstoffatom an das sie gebunden sind, einen gesättigten oder ungesättigten gegebenenfalls benzokondensierten drei- bis siebengliedrigen Ring, der bis zu drei Heteroatome aus der Gruppe O, N und S enthält und der unsubstituiert oder durch ($C_1$-$C_4$)-Alkyl oder Halogen substituiert ist und eine Carbonylgruppe enthalten kann,

$R^8$= jeweils unabhängig voneinander H, unsubstituiertes oder durch Phenyl, Halogenphenyl, Nitrophenyl, Cyanophenyl, ($C_1$-$C_4$)-Alkylphenyl oder ($C_1$-$C_4$)-Alkoxyphenyl, Hydroxy, Cyano, ($C_1$-$C_4$-Alkoxy)-carbonyl, ($C_1$-$C_4$)-Alkylthio, ($C_1$-$C_4$)-Alkoxy, Cyclo-($C_5$-$C_7$)alkyl oder Benzyloxy substituiertes ($C_1$-$C_{12}$)-Alkyl, Cyclo-($C_5$-$C_8$)alkyl, ($C_3$-$C_6$)-Alkenyl, Halogen-($C_3$-$C_6$)alkenyl, ($C_3$-$C_6$)-Alkinyl, Cyclo-($C_5$-$C_6$)alkenyl, ($C_1$-$C_6$-Alkyl)-carbonyl, Halogen-($C_1$-$C_6$-alkyl)carbonyl mit 1 bis 3 Halogenatomen, ($C_1$-$C_6$-Alkylamino)carbonyl, Benzoyl, Halogenbenzoyl oder Methylbenzoyl,

$R^9$= jeweils unabhängig voneinander H, Halogen, ($C_1$-$C_4$)-Alkyl, Nitro oder Cyano,

$R^{10}$= unabhängig voneinander $(C_1-C_6)$-Alkyl, Cyclo-$(C_3-C_6)$-alkyl, $(C_3-C_6)$-Alkenyl, Phenyl, Benzyl oder beide Reste $R^{10}$ gemeinsam mit dem Kohlenstoff, an das sie gebunden sind, ein unsubstituiertes oder durch Methyl oder Halogen substituiertes Cyclo-$(C_5-C_7)$alkyl,

$R^{11}$= $(C_1-C_4)$-Alkyl, Phenyl, $(C_1-C_6$-Alkyl)carbonyl, Benzyl, Benzoyl, Halogenbenzyl, Halogenbenzoyl oder Methylbenzoyl und

$R^{12}$= H, $(C_1-C_4)$-Alkyl, Formyl, $(C_1-C_6$-Alkyl)carbonyl, Benzoyl, Halogenbenzoyl, Methylbenzoyl oder Trihalogenacetyl, bedeuten

sowie deren für die landwirtschaftlichen Zwecke verträglichen Salze und Quaternisierungsprodukte, mit der Maßgabe, daß nur ein Rest $R^1$ $(C_1-C_4)$-Alkyl sein darf. Die Salzbildung bzw. Quaternisierung erfolgt hierbei durch Addition geeigneter Verbindungen an die -S-$R^3$-Gruppe oder an das basische Stickstoffatom des Imidazolrings. Die Salzbildung oder Quaternisierung ist nicht möglich, wenn $R^4$, $R^5$ ein Kation bedeutet, X für -SH steht oder $R^4$, $R^5$ eine Carboxylatgruppe enthält.

Für den Fall, daß in den aufgeführten Substituenten - zusätzlich zum Imidazolring - weitere basische Stickstoffatome auftreten, ist auch eine mehrfache Salzbildung oder Quaternisierung möglich.

Zur Salzbildung am Stickstoff geeignet sind alle anorganischen oder organischen Säuren, die aufgrund ihres pKs-Wertes zur Salzbildung befähigt sind, z.B. Halogenwasserstoffsäuren, Salpetersäure, Schwefelsäure, Phosphorsäure, Phosphonsäure, Sulfonsäuren, Halogenessigsäuren oder Oxalsäure.

Als Sulfoniumsalze (Salzbildung am Schwefel) oder Quaternisierungsprodukte (Salzbildung am Stickstoff) sind die Umsetzungsprodukte mit Alkyl-, Alkylthioalkyl, Alkoxyalkyl-, insbesondere ($C_1$-$C_6$)-Alkyl- und gegebenenfalls im Phenylrest substituierten, insbesondere halogenierten Phenacylhalogeniden zu verstehen. Die Herstellung der Salze und Quaternisierungsprodukte der Verbindungen der Formel I erfolgt nach allgemein üblichen Methoden.

Als Acetale, Ketale und Thioketale kommen insbesondere solche der Formel $-C(OR^{13})_2R^6$ oder $-C(SR^{13})_2R^6$ in Betracht worin $R^{13}$= ($C_1$-$C_2$)-Alkyl oder beide Reste $R^{13}$ zusammen eine $C_2$- oder $C_3$-Alkylenkette bedeuten, die durch ($C_1$-$C_4$)-Alkyl, Monohydroxy-($C_1$-$C_4$)-alkyl, Halogen-($C_1$-$C_4$)alkyl mit 1 bis 3 Halogenatomen oder Phenyl substituiert sein kann.

Unter den Heterocyclen-Ringen für $-NR^6R^7$ kommen insbesondere infrage Piperidin, Morpholin, 2,6-Dimethylmorpholin, Piperazin, Imidazol, Thiazol oder Benzimidazol. Die bei der Definition der allgemeinen Formel (I) vorkommenden Alkyl-, Alkenyl- und Alkinylreste können sowohl geradkettig als auch verzweigt sein.

Unter Halogen ist F, Cl, Br oder J, insbesondere F, Cl oder Br zu verstehen. Im Falle $R^1$ bedeutet fluoriertes Alkyl, bevorzugt perfluoriertes Alkyl und ganz besonders Trifluormethyl. Halogeniertes ($C_3$-$C_6$)-Alkenyl enthält insbesondere 1 bis 3 Brom-, Chlor- oder Fluoratome.

Halogenphenyl, Halogenbenzyl oder Halogenbenzoyl enthalten insbesondere 1 bis 3 Fluor-, Chlor- oder Bromatome.

Unter Trihalogenacetyl ist insbesondere Trichlor- und Trifluoracetyl zu verstehen.

Als Kationen für $R^4$ und $R^5$, die für die Landwirtschaft einsetzbar sind, kommen Metallkationen z.B. Alkali- oder

Erdalkalikationen wie Na, K, Mg oder organische Kationen wie organisch substituiertes Ammonium organisch substituiertes Phosphonium, Sulfonium oder Sulfoxonium oder andere Stickstoff-Kationen in Betracht.

Organisch substituiertes Ammonium bedeutet primäres, sekundäres, tertiäres, quartäres, aliphatisches, aromatisches oder heteroaromatisches Ammonium, das 1 bis 3 N-Atome enthalten kann. Die Stickstoffatome des Amins können hierbei auch Teil eines cyclischen Systems sein. Als Beispiele für solche Ammoniumsalze seien genannt: Mono-, Di-, Tri-, Tetra-[($C_1$-$C_6$)-Alkyl]ammonium wie Isopropylammonium, Butylammonium, Stearylammonium Trietyhylammonium, Mono-, Di-, Tri-[($C_1$-$C_4$)alkyl($C_1$-$C_4$)-alkyl]ammonium oder Mono-, Di-, Tri-[($C_1$-$C_6$)alkanol]-ammonium wie Methoxyethylammonium, Methoxypropylammonium, Triethanolammonium, Tripropanolammmonium oder Ammoniumverbindungen mit gemischten Resten wie tert.-Butyldiethanolammonium, Triethylbenzylammonium, Hydroxyethyltrimethylammonium, Chlorethyltrimethylammonium; oder Allylammonium, Diallylammonium, Cyclohexylammonium, Menthylammonium, Aminoethylammonium, Ethylendiammonium, Benzhydrylammonium, Pyrrolidinium, Morpholinium, 3-Piperidinium oder Piperazinium, oder ein von einer Aminosäure oder deren Ester abgeleitetes Ammonium wie $NH_3$-$CH_2$-$COOCH_3$.

Entsprechend enthalten organisch substitutiertes Phosphonium, organisches Sulfonium oder organisches Sulfoxonium aliphatische oder arylaliphatische Reste.

Andere Stickstoff-Kationen sind beispielsweise Hydrazonium, Hydroxylammonium, Guanidinium, Aminoguanidinium oder deren Substitutionsprodukte.

Bevorzugt unter den Verbindungen der Formel I

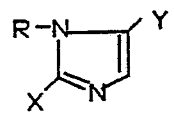

I

sind solche, für die

R = einen Rest der Formel

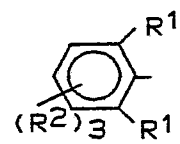

X = einen Rest der Formeln -SH,

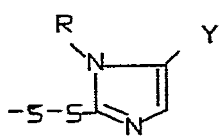

oder Wasserstoff

Y = einen Rest der Formeln

$-CN$, $-\overset{\overset{O}{\|}}{C}-OR^4$, $-\overset{\overset{O}{\|}}{C}-SR^5$, $-\overset{\overset{O}{\|}}{C}-NR^6R^7$, $-\overset{\overset{O}{\|}}{C}-R^6$, $-CF_3$,

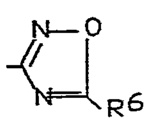 , 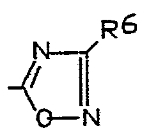 , 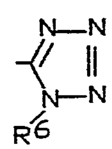 ,

$R^1$= unabhängig voneinander ($C_1$-$C_3$)-Alkyl, das halogeniert sein kann, ($C_2$-$C_3$)-Alkenyl, ($C_2$-$C_3$)-Haloalkenyl, Acetyl, ($C_1$-$C_3$)-Hydroxyalkyl, ($C_1$-$C_4$)-Alkoxy oder Halogen,

$R^2$= unabhängig voneinander Wasserstoff, ($C_1$-$C_4$)-Alkyl oder Halogen,

$R^4$= Wasserstoff, $(C_1-C_6)$-Alkyl, $(C_1-C_6)$-Alkyl, das ein- bis dreifach durch Hydroxy, Halogen, $(C_1-C_6)$-Alkoxy, $(C_1-C_4)$-Alkylthio, Cyano, Trimethylsilyl, Benzyloxy oder Phenyl substituiert ist, $(C_3-C_6)$-Alkenyl, halogeniertes $(C_3-C_6)$-Alkenyl, Cyclo-$(C_3-C_7)$alkyl, $(C_3-C_6)$-Alkinyl, Phenyl oder einen Rest der Formeln $-N=C(R^{10})_2$,

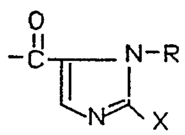,

oder ein für die Landwirtschaft einsetzbares Kation,

$R^5$= H, $(C_1-C_6)$-Alkyl,

$R^6$= Wasserstoff, $(C_1-C_6)$-Alkyl,

$R^7$= Wasserstoff, $(C_1-C_6)$-Alkyl oder $(C_1-C_6)$-Alkyl, das bis zu zweifach, durch $(C_1-C_2)$-Alkoxy substituiert ist, Phenyl, $(C_3-C_6)$-Alkenyl, $(C_3-C_6)$-Cycloalkyl, einen Rest der Formeln $-NR^6R^{12}$, $-OR^6$,

$R^{10}$= unabhängig voneinander $(C_1-C_4)$-Alkyl,

$R^{12}$= H, $(C_1-C_4)$-Alkyl, bedeuten

sowie deren für die landwirtschaftlichen Zwecke verträglichen Salze und Quaternisierungsprodukte, mit der Maßgabe, daß nur ein Rest $R^1$ $(C_1-C_4)$-Alkyl sein darf.

Besonders bevorzugt unter den Verbindungen der Formel I sind solche,

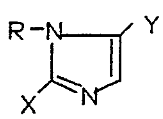

I

worin

R = einen Rest der Formel

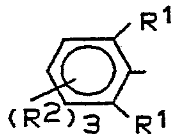

X = Wasserstoff

Y = $-\underset{\underset{O}{\|}}{C}-OR^4$, $-\underset{\underset{O}{\|}}{C}-NR^6R^7$ oder $-\underset{\underset{O}{\|}}{C}-H$

$R^1$= unabhängig voneinander ($C_1$-$C_3$)-Alkyl, Trifluormethyl, Vinyl, ($C_1$-$C_4$)-Alkoxy, Fluor oder Chlor,

$R^2$= unabhängig voneinander Wasserstoff, Methyl oder Chlor,

$R^4$= Wasserstoff, ($C_1$-$C_6$)-Alkyl, ($C_1$-$C_6$)-Alkyl, das ein- bis dreifach durch Halogen oder einfach durch Trimethylsilyl substituiert ist, Allyl, Propargyl, einen Rest der Formeln $-N{=}C(R^{10})_2$,

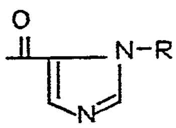

oder ein für die Landwirtschaft einsetzbares Kation,

$R^6$= Wasserstoff, ($C_1$-$C_6$)-Alkyl,

$R^7$= Wasserstoff, ($C_1$-$C_6$)-Alkyl oder ($C_1$-$C_6$)-Alkyl, das bis zu zweifach durch Methoxy, Ethoxy substituiert ist, Allyl, ($C_3$-$C_6$)-Cycloalkyl, einen Rest der Formeln $-NR^6R^{12}$ oder $-OR^6$,

$R^{10}$= $(C_1-C_4)$-Alkyl,

$R^{12}$= Wasserstoff oder Methyl, bedeuten

sowie deren für die landwirtschaftlichen Zwecke verträglichen Salze und Quaternisierungsprodukte, mit der Maßgabe, daß nur ein Rest $R^1$ $(C_1-C_3)$-Alkyl sein darf.

Gegenstand der Erfindung ist ferner ein Verfahren zur Herstellung der Verbindungen der Formel I und ihrer Salze oder Quaternisierungsprodukte, dadurch gekennzeichnet, daß man

a) einen Bisformylester der Formeln IIa oder IIb

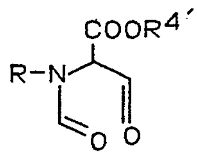

IIa

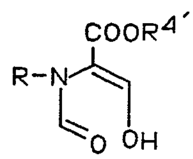

IIb

wobei $R^{4'}$= $(C_1-C_{12})$-Alkyl bedeutet,

$(a_1)$ mit einem $(C_1-C_3)$-Carbonsäureamid cyclisiert oder

$(a_2)$ mit einem Alkali- oder Ammoniumrhodanid zu einem 2-Mercaptoimidazol-Derivat umsetzt und dieses gegebenenfalls mit Salpetersäure und Natriumnitrit entschwefelt

und die erhaltenen Verbindungen derivatisiert;

b) eine Imidazolverbindung der Formel III,

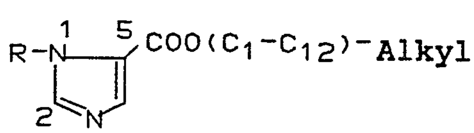

III

mit einer starken Base in 2-Stellung des Imidazolrings metalliert und anschließend mit einem Disulfid der Formel $R^3$-S-S-$R^3$ zu einer Verbindung der Formel I mit X = -S-$R^3$ und Y = COO($C_1$-$C_{12}$-alkyl) umsetzt, und die erhaltenen Verbindungen derivatisiert.

c) Eine Imidazolverbindung der Formel IV

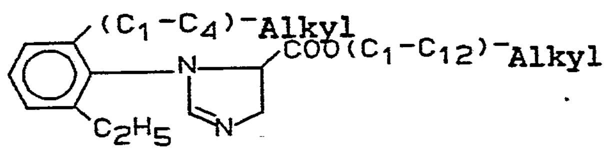

IV

an der Ethylgruppe oxidiert und so eine Verbindung der Formel I mit $R^1$ = Acetyl erhält und die erhaltenen Verbindungen ggf. derivatisiert,

d) eine Iminoverbindung der Formeln Va oder Vb

R-N=CH-COO($C_1$-$C_4$)-Alkyl Va

R-N=CH-$CF_3$ Vb

mit p-Toluolsulfonylmethylisocyanid umsetzt und die erhaltenen Verbindungen der Formel I gegebenenfalls derivatisiert.

Die Derivatisierung der Verbindungen bei denen X nicht für Wasserstoff steht kann in bekannter Weise, wie dies z.B. in der Patentanmeldung DE-A 35 37 290 beschrieben ist, entweder aus dem 2-Mercapto-imidazol (Verfahrensvariante $a_2$) oder durch Umsetzung des Imidazols mit X = H mit Disulfiden in Gegenwart starker Basen erfolgen.

Bei der Derivatisierung des Substituenten in 5-Stellung des Imidazols wird der Rest $-COOR^4$, in bekannter Weise modifiziert z.B. durch Verseifung, Veresterung, Umesterung, Amidierung, Salzbildung, Reduktion oder Oximierung wie dies z.B. in den Patentanmeldungen DE-A 34 44 918 und DE-A 34 42 690 beschrieben ist, oder es erfolgt Salzbildung oder Quaternisierung am basischen Stickstoffatom des Imidazolringes.

Im Verfahren ($a_1$) wird als Carbonsäureamid vorzugsweise Formamid eingesetzt. Es wird bevorzugt in Gegenwart von Mineralsäure in molaren Mengen bei 50 - 200°C, insbesondere 100 - 170°C umgesetzt.

Die Bisformylverbindungen der Formel IIa bzw. IIb lassen sich leicht nach bekannten Verfahren (DE-OS 32 17 094) aus den bekannten Anilinen (z.B. 2-Alkyl-6-trifluormethylaniline US-Patent 45 03 276; 2-Acetyl-, 2-Alkenyl-, 2-Hydroxyalkyl-, 6-alkylaniline US-Patent 44 56 471; 2-Alkoxy-6-alkylaniline analog Gibson, Soc. 123, 1269; 2-Methyl-6-halogenaniline analog P. Claus und W. Vycndilik, Tetrahedron Lett., 1968, 3610) erhalten.

Die Iminoverbindungen der Formeln Va und Vb lassen sich leicht aus den bekannten vorstehenden Anilinen und Glyoxylsäureestern bzw. Trifluoracetaldehyd herstellen. Die Cyclisierung zu den Imidazolen der Formel I erfolgt in bekannter Weise (vgl. VA. van Lensen et al. J. Org. Chem., 42, 1153, (1977)) in guter Ausbeute.

Die Phenylimidazolverbindungen IV sind ebenfalls literaturbekannt, s. DE-OS 32 17 094. Die Ethylgruppe läßt sich mit geeigneten Oxidationsmitteln (z.B. $KMnO_4$, pH 3 - 5) zur Acetylgruppe oxidieren. Die Acetylgruppe kann dann auf bekannte Weise, z.B. durch Natriumborhydrid-Reduktion zur 1-Hydroxyethylgruppe durch anschließende Wassereliminierung zur Vinylgruppe, derivatisiert werden.

Verbindungen der Formel I mit $R^1$ = Alkenyl lassen sich auf übliche Weise z.B. durch Chlor- oder Bromaddition in Halogenalkylverbindungen und durch anschließende Eliminierung in Halogenalkenylverbindungen überführen.

Die Salze der Verbindungen der Formel (I) können in einfacher Weise nach üblichen Salzbildungsmethoden, z.B. durch Lösen einer Verbindung der Formel (I) in einem geeigneten organischen Lösemittel und Hinzufügen der Säure erhalten werden und in bekannter Weise, z.B. durch Abfiltrieren, isoliert und gegebenenfalls durch Waschen mit einem inerten organischen Lösemittel gereinigt werden.

Mit den erfindungsgemäßen Verbindungen sind typische wachstumsregulierende Effekte erzielbar, die - verglichen den aus der DE-A 32 17 094 bekannten Verbindungen - in verschiedenen Kulturen bereits bei niederen Dosierungen eingesetzt werden können. Sie greifen regulierend in den pflanzeneigenen Stoffwechsel ein und können damit zur gezielten Beeinflussung von Pflanzeninhaltsstoffen sowie zur Ernteerleichterung wie zum Auflösen von Desikkation und Wuchsstauchung eingesetzt werden. Des weiteren eignen sie sich zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann. Besonders hervorzuheben ist die wachstumsregulatorische Wirksamkeit der Verbindungen als Wuchshemmer in Getreide, Mais, Soja, Tabak, Baumwolle, Ackerbohne, Raps, Reis, Rasen sowie ihre Fähigkeiten, den Gehalt an erwünschten Inhaltsstoffen wie Kohlehydraten (z.B. Zuckerrohr oder Hirsekulturen) und Protein bei Nutzpflanzen zu erhöhen. Schließlich zeigen die Verbindungen eine sehr gute Verbesserung der Fruchtabszission, insbesondere bei Zitrusfrüchten.

Die Mittel können als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Ein weiterer Gegenstand der Anmeldung sind auch Pflanzenschutzmittel, die sich durch einen wirksamen Gehalt mindestens einer Verbindung der allgemeinen Formel (I) auszeichnen.

Die erfindungsgemäßen Verbindungen lassen sich bei ihrem praktischen Einsatz gegebenenfalls auch vorteilhaft mit bekannten Wachstumregulatoren kombinieren. Solche bekannten Wachstumsregulatoren sind die Verbindungen der Formel

$$R\text{-}CH_2\text{-}CH_2\text{-}N^+(CH_3)_3Cl^- \qquad (VI)$$

worin R = OH oder Cl bedeutet (common name Chlormequat für R = Cl), ferner N,N-Dimethylpiperidiniumchlorid (VII, Mepiquat-Chlorid), α-Cyclopropyl-4-methoxy-α-(pyrimidin-5-yl)-benzylalkohol (VIII, Ancymidol), 3aα, 4ß, 4aα, 6aα, 7ß, 7aα(-1-(4-chlorophenyl)-3a, 4, 4a, 6a, 7, 7a-hexahydro-4,7-methano-1H-[1,2]-diazeto-[3,4-f]-benzotriazol (IX, Tetcyclacis), Bernsteinsäure-mono-2,2-dimethylhydrazid (X, Diaminoazide), 6-Hydroxy-2H-pyridazin-3-on (XI, Maleinsäurehydrazid), 2-Chlor-9-hydroxy-9H-fluoren-9-carbonsäure (XII, Chlorflurenol), 5'-(Trifluormethan-sulfonamido)-acetat-2', 4'-xylidid (XIII, Mefluidid), 2-Chlorethylphosphonsäure (XIV, Ethephon).

Die wachstumsregulatorischen Wirkungen der Verbindungen der Formeln (VI) bis (XIV) sind beschrieben in Plant Growth Regulator Handbook of the Plant Growth Regulator Working Group 2d. Ed. 1981.

Anstelle der Verbindungen der Formeln (VIII) und (VII) können prinzipiell auch vergleichbare Salze, die anstelle

des Chloridions ein anderes übliches Anion wie Bromid, Nitrat oder 1/2 Sulfat enthalten, eingesetzt werden.

Bei der Kombination der Verbindungen der Formel (I) mit den Verbindungen der Formeln (VI) bis (XIV) zeigten sich überraschenderweise auffallende synergistische Wirkungen. So lassen sich diese Kombinationen in noch geringeren Dosierungen einsetzen, als von der Wirkung der Einzelkomponenten zu erwarten war, um die gewünschten Effekte zu erzielen. Mit den Kombinationen lassen sich auch Wildwuchserscheinungen vermindern, so daß die Kombinationen auch in der Landschaftspflege eingesetzt werden können. Desweiteren eignen sich die Kombinationen hervorragend zur generellen Steuerung und Hemmung von unerwünschtem vegetativen Wachstum, wie Seitentriebbildung, ohne die Pflanzen abzutöten. Die Verbindungen der Formel (I) können auch vorteilhaft mit zwei verschiedenen Verbindungen der Formeln (VI) bis (XIV) kombiniert werden.

Die Mischungsverhältnisse der Komponenten der allgemeinen Formel (I) zu den Verbindungen der Formeln (VI) bis (XIV) können innerhalb weiter Grenzen, etwa zwischen 250 : 1 bis 1 : 10, schwanken. Die Wahl der Mischungsverhältnisse ist abhängig von der Art der Mischungspartner, vom Entwicklungsstadium der Pflanzen als auch vom Grad der gewünschten wachstumsregulatorischen Wirkung. Vorzugsweise werden Mischungsverhältnisse von 10 : 1 bis 1 : 10 gewählt.

Die Kombinationen können sowohl als Mischformulierungen der Komponenten vorliegen, die dann in üblicher Weise mit Wasser verdünnt zur Anwendung gebracht werden; oder sie können als sogenannte Tankmischungen durch gemeinsame Verdünnung der getrennt formulierten Komponenten mit Wasser hergestellt werden; es besteht auch die Möglichkeit, die Komponenten nacheinander zur Anwendung zu bringen, d.h. es werden dann die Komponenten in Einzelformulierungen appliziert.

Die Verbindungen der allgemeinen Formel (I) können auch mit natürlichen oder pflanzlichen Hormonen wie Auxinen oder Cytokinen kombiniert werden.

Die erfindungsgemäßen Verbindungen der allgemeinen Formel (I), können, gegebenenfalls im Gemisch mit weiteren Wirkkomponenten, wie den Verbindungen der Formeln VI bis XII, als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Spritzpulver sind in Wasser gleichmäßig dispergierbare Präparate, die neben dem (den) Wirkstoff(en) außer gegebenenfalls einem Verdünnungs- oder Inertstoff noch Netzmittel wie polyoxethylierte Fettalkohole, Alkyl- oder Alkylphenylsulfonate und/oder Dispergierhilfsmittel wie ligninsulfonsaures Natrium, 2,2'-dinaphthylmethan-6,6'-disulfonsaures Natrium, dibutylnaphthalinsulfonsaures Natrium oder auch oleoylmethyltaurinsaures Natrium enthalten. Die Herstellung erfolgt in üblicher Weise, z.B. durch Mahlen und Vermischen der Komponenten.

Emulgierbare Konzentrate können z.B. durch Auflösen des (der) Wirkstoff(e) in einem inerten organischen Lösemittel wie Butanol, Cyclohexanon, Dimethylformamid, Xylol oder auch höhersiedenden Aromaten oder aliphatischen oder cycloaliphatischen Kohlenwasserstoffen unter Zusatz von einem oder mehreren Emulgatoren hergestellt werden. Bei flüssigen Wirkstoffen kann der Lösemittelanteil auch ganz oder teilweise entfallen. Als Emulgatoren können beispielsweise verwendet werden: Alkyl-arylsulfonsaure Calciumsalze wie Ca-dodecylbenzolsulfonat oder nicht ionische Emulgatoren wie Fettsäurepolyglykolester, Alkylarylpolyglykolether, Fettalkoholpolyglykolether, Propylenoxid-Ethylenoxid-Kondensationsprodukte, Fettalkohol-Propylenoxid-Kondensationsprodukte, Alkylpolyglykolether, Sorbitanfettsäureester, Polyoxethylensorbitanfettsäureester oder Polyoxethylensorbitester.

Stäubemittel kann man durch Vermahlen des (der) Wirkstoffe(s) mit fein verteilten, festen Stoffen, z.B. Talkum, natürlichen Tonen wie Kaolin, Bentonit, Pyrophillit oder Diatomeenerde erhalten. Granulate können entweder durch Verdüsen der (des) Wirkstoffe(s) auf adsorptionsfähiges, granuliertes Inertmaterial hergestellt werden oder durch Aufbringen von Wirkstoffkonzentraten mittels Bindemitteln, z.B. Polyvinylalkohol, polyacrylsaurem Natrium oder auch Mineralölen auf die Oberfläche von Trägerstoffen wie Sand, Kaolinite oder von granuliertem Intertmaterial. Auch können geeignete Wirkstoffe in der für die Herstellung von Düngemittelgranulaten üblichen Weise - gewünschtenfalls in Mischung mit Düngemitteln - granuliert werden.

In Spritzpulvern beträgt die Wirkstoffkonzentration etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 0,05 bis 20 Gew.-% an Wirkstoff(en), versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden. Daneben enthalten die genannten Wirkstofformulierungen gegebenenfalls die jeweils üblichen Haft-, Netz-, Dispergier-, Emulgier-, Penetrations-, Lösemittel, Füll- oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten,

Dispersionen und teilweise auch bei Mikrogranulaten mittels Wasser. Staubförmige und granulierte Zubereitungen sowie versprühbare Lösungen werden vor der Awendung üblicherweise nicht mehr mit weiteren inerten Stoffen verdünnt.

Die Aufwandmengen der Wirkstoffe der Formel I, können in weiten Grenzen variieren. Sie liegen im allgemeinen zwischen 0,02 und 1,5 kg Wirkstoff pro ha, vorzugsweise zwischen 0,05 bis 1 kg/ha.

**Chemische Beispiele**

**Beispiel 1**

**Ethyl-1-(2-ethyl-6-trifluormethyl-phenyl)-imidazol-5-carboxylat**

1,3 g (3,9 mmol) Ethyl-2-(N-formyl-2-ethyl-6-trifluormethyl-anilino)-3-hydroxy-acrylat wurden mit 20 ml Formamid und 5 ml konzentrierter Salzsäure 4 h auf 150°C erhitzt. Nach dem Erkalten extrahierte man zweimal mit Diisopropylether, wusch die organische Phase mit Wasser, trocknete über Natriumsulfat und dampfte ein. Nach chromatographischer Reinigung erhielt man 0,7 g (57 % der Theorie) Ethyl-1-(2-ethyl-6-trifluormethyl-phenyl)-imidazol-5-carboxylat, ein farbloses Öl. Die Identifizierung erfolgte NMR-spektroskopisch.

Nach dem vorstehend beschriebenen Verfahren lassen sich die folgenden Beispiele herstellen.

Stehen in den Tabellen 1 und 2 für Y andere Reste als $-COOCH_3$ oder $COOC_2H_5$ wurden diese nach gängigen, allgemein bekannten Verfahren aus den entsprechenden Estern hergestellt. Die Derivate bei denen X nicht für Wasserstoff steht, wurden analog in der Deutschen Offenlegungsschrift 35 27 290 beschriebenen Verfahren hergestellt.

BAD ORIGINAL

Tabelle 1 Imidazolverbindungen der Formel I, worin ein Rest $R^1 = CF_3$ ist.

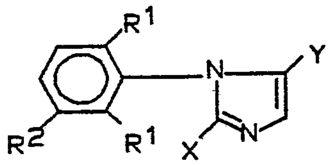

I

| Bsp. Nr. | $R^1$ | $R^2$ | X | Y | Fp/Sdp. [°C] |
|---|---|---|---|---|---|
| 2 | $CH_3$ | H | H | $-COOH$ | |
| 3 | " | " | " | $-COOCH_3$ | |
| 4 | " | " | " | $-CN$ | |
| 5 | " | " | " | $-CONH_2$ | |
| 6 | " | " | " | $-C(=NOH)NH_2$ | |
| 7 | " | " | " | $-CHO$ | |
| 8 | " | " | " | $-CH(SC_2H_5)_2$ | |
| 9 | " | " | SH | $COOC_6H_{13}$ | |
| 10 | " | " | $SCH_3$ | $COOC_6H_{13}$ | |
| 11 | " | " | $S(=O)CH_3$ | $CON(CH_3)$Phenyl | |
| 12 | " | " | $A(=O)_2CH_3$ | 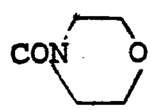 | |
| 13 | " | 3-Cl | H | $-COO^- \cdot NH_4^+$ | |
| 14 | " | " | " | $-COO^- \cdot N_3N^+-CH(-CH_3)_2$ | |
| 15 | " | " | " | $-COO \cdot 1/2Mg$ | |
| 16 | " | " | " | $-C(=O)NHOH$ | |
| 17 | " | $4-CH_3$ | " | 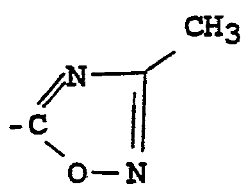 | |
| 18 | " | " | " | $-CONH-(CH_2)_2-OH$ | |
| 19 | $C_2H_5$ | H | H | $COOH$ | |
| 20 | " | " | " | $COONa$ | |
| 21 | " | " | " | $COOK$ | |
| 22 | " | " | " | $COO^- \cdot H\overset{+}{N}(-CH_2CH_2-OH)_3$ | |

Fortsetzung Tabelle 1

| Bsp. Nr. | $R^1$ | $R^2$ | X | Y | Fp/Sdp. [°C] |
|---|---|---|---|---|---|
| 23 | $C_2H_5$ | H | H | $COO^- \cdot H_2N^+$ 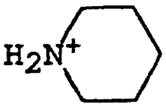 | |
| 24 | " | " | " | COOH | (Hydrochlorid) |
| 25 | " | " | " | $COOCH_3$ | |
| 26 | " | " | " | $COOCH(-CH_3)_2$ | |
| 27 | " | " | " | $COO-N=C(CH_3)_2$ | |
| 28 | " | " | " | $COOCH_2CF_3$ | |
| 29 | " | " | " | $COOCH_2CH_2Cl$ | |
| 30 | " | " | " | $COO(CH_2)_3-N(CH_3)_2$ | |
| 31 | " | " | " | $-CONH_2$ | |
| 32 | " | " | " | $-CONH-CH(OCH_3)_2$ | |
| 33 | " | " | " | $-CON(CH_3)CH_2-CH(OC_2H_5)_2$ | |
| 34 | " | " | " | -CON 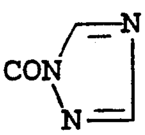 | |
| 35 | " | " | " | -CONH- 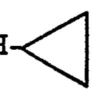 | |
| 36 | " | " | " | -CONH- 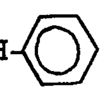 | |
| 37 | " | " | " | -CONH- 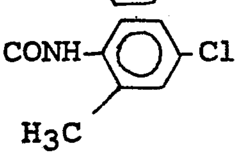 | |
| 38 | " | " | " | $-COOCH_2-CH=CH_2$ | |
| 39 | " | " | " | $-COO-CH_2-C≡CH$ | |
| 40 | " | " | " | $-C(=S)-NH_2$ | |
| 41 | " | " | " | $COSC_2H_5$ | |

Fortsetzung Tabelle 1

| Bsp. Nr. | $R^1$ | $R^2$ | X | Y | Fp/Sdp. [°C] |
|---|---|---|---|---|---|
| 42 | $C_2H_5$ | H | SH | COOH | |
| 43 | " | " | S-$CH_2$-Phenyl | " | |
| 44 | " | 4-Br | -S-Phenyl | $COOCH_2$-$COOCH_3$ | |
| 45 | " | " | H | $CH_2OH$ | |
| 46 | " | " | " | $CH_2$-O-$COCH_3$ | |
| 47 | " | " | " | CONH-N$(CH_3)_2$ | |
| 48 | " | " | " | CONH-O-Benzyl | |
| 49 | " | " | " | CONH-$OCH_3$ | |
| 50 | " | " | " | 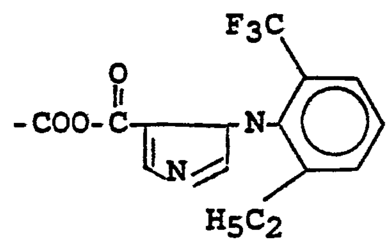 | |
| 51 | " | " | " | -COO-Phenyl | |
| 52 | " | " | " | -COO-$CH_2CH_2$-$OCH_3$ | |
| 53 | " | " | " | -CONH-$CH_2$-CN | |
| 54 | " | " | " | -$COOCH_2$-S-$CH_3$ | |
| 55 | " | " | " | -COOLi | |
| 56 | " | " | " | -CH=NOH | |
| 57 | " | " | " | -CH=$NOCH_3$ | |
| 58 | $CH(CH_3)_2$ | " | " | -COOH | |
| 59 | " | " | " | 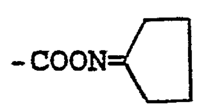 | |
| 60 | " | " | -SH | -$COOC_{12}H_{25}$ | |
| 61 | " | " | -$SCH_2COOCH_3$ | -$CONHC_8H_{17}$ | |
| 62 | " | " | 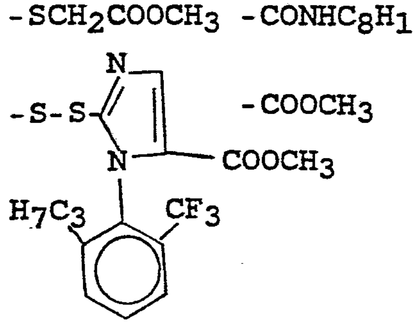 | -$COOCH_3$ | |

Beispiel 63

**1-(2-Isopropenyl-6-methyl-phenyl)-imidazol-5-carbonsäure**

8,0 g (0,03 mol) Ethyl-1-(2-isopropenyl-6-methyl-phenyl)-imidazol-5-carboxylat (s. Beispiel 128) werden mit 100 ml 2-N-Natronlauge 2 Stunden auf 80°C erhitzt. Man läßt abkühlen, säuert mit konzentrierter Salzsäure auf pH 3 an und saugt den Niederschlag ab. Man erhält 5,2 g (72 % d. Theorie) 1-(2-Isopropenyl-6-methyl-phenyl)-imidazol-5-carbonsäure, ein farbloser Feststoff vom Schmelzpunkt 204°C (Zersetzung).

Beispiel 64

**Ethyl-1-(2-(1,2-Dibromisopropyl-6-methylphenyl)-imidazol-5-carboxylat**

Zu 10,0 g (0,037 mol) Ethyl-1-(2-isopropenyl-6-methylphenyl)-imidazol-5-carboxylat werden bei Raumtemperatur in 100 ml Tetrachlorkohlenstoff 6,0 g (0,037 mol) Brom zugetropft. Nach 1 Stunde wird in 200 ml Natriumcarbonatlösung gegossen, die organische Phase abgetrennt, mit Wasser gewaschen und im Vakuum eingedampft. Man erhält 14,3 g (89 % d. Theorie) Ethyl-1-(2-[1,2-Dibromisopropyl ]-6-methyl-phenyl)-imidazol-5-carboxylat, ein Feststoff vom Schmelzpunkt 103 - 105°C.

$^1H$-NMR

(60 MHz, $CDCl_3$) $\delta$ = 1,07 (t, J = 7 Hz, 3H, $-O-CH_2-\underline{CH_3}$), 1,83 (s, 3H, Phenyl-$\underline{CH_3}$), 1,91, 1,98 (2s, zus. 3H = CBr-$\underline{CH_3}$), 3,8 - 4,4 (sh, 4H, -$\underline{OCH_2}$, -CBr$\underline{H_2}$), 7,1 - 7,5 (m, 3H-Phenyl-$\underline{H}$), 7,7, 7,8 (2s, zus. 1H, Imidazol-$\underline{H}$), 7,9 (s, 1H, Imidazol-$\underline{H}$) ppm.

Beispiel 65

**Ethyl-1-(2-(1-bromisoprop-1-enyl-6-methylphenyl)-imidazol-5-carboxylat**

8,3 g (0,019 mol) Ethyl-1-(2-(1,2-Dibromisopropyl-6-methyl-phenyl)-imidazol-5-carboxylat (s. Beispiel 64) werden in

Gegenwart einer Natriumethylatlösung (hergestellt aus 1,0 g Natrium (0,043 mol) und 50 ml Ethanol) 3 Stunden unter Rückfluß erhitzt. Man läßt abkühlen, nimmt in Toluol/Wasser auf, trennt die organische Phase ab, trocknet sie über Natriumsulfat und dampft ein. Man erhält 6,3 g (95 % d.Th.) Ethyl-1-(2-(1-bromisoprop-1-enyl-6-methylphenyl)-imidazol-5-carboxylat, ein hellgelber Feststoff vom Schmelzpunkt 97 - 99°C.

**Beispiel 66**

**Ethyl-1-(2-acetyl-6-ethylphenyl)-imidazol-5-carboxylat**

Zu einer Lösung von 21,8 g (0,080 mol) Ethyl-1-(2,6-diethylphenyl)-imidazol-5-carboxylat und 121 g Magnesiumnitrat in 300 ml tert.-Butanol tropft man bei 20 - 30°C eine Lösung von 27,3 g (0,13 mol) Kaliumpermanganat in 1000 ml Wasser zu. Man rührt 1 Stunde bei 50°C nach, saugt bei dieser Temperatur vom Braunstein ab, extrahiert die Mutterlauge 2mal mit Toluol, trocknet die organische Phase (Natriumsulfat), dampft ein und chromatographiert den Rückstand über eine Kieselgelsäule (Petrolether-Essigester 9:1 bis 1:1).

Man erhält neben 11 g Edukt 8,6 g (75, % d. Th. bezgl. umgesetztem Edukt) Ethyl-1-(2-acetyl-6-ethylphenyl)-imidazol-5-carboxylat, ein hellgelbes Öl.

**$^1$H-NMR**

(60 MHz, $CDCl_3$) $\delta$ = 1,07 (t, J = 7 Hz, 3H, Phenyl-$CH_2$-$\underline{CH_3}$), 1,2 (t, J = 7 Hz, 3H, -O-$CH_2$-$\underline{CH_3}$), 2,23 (s, 3H, $COCH_3$), 2,31 (q, J = 7 Hz, 2H, Phenyl-$\underline{CH_2}$), 4,10 (q, J = 7 Hz, 2H, O-$CH_2$), 7,3 - 7,6 (sh, 4H, Phenyl-$\underline{H}$, Imidazol-$\underline{H}$), 7,83 (s, 1H, Imidazol-$\underline{H}$) ppm.

**Beispiel 67**

**Ethyl-1-(2-ethyl-6-vinylphenyl)-imidazol-5-carboxylat**

10,6 g (0,04 mol) Ethyl-1-(2-ethyl-6-(1-hydroxy)-ethyl-

phenyl)-imidazol-5-carboxylat (s. Beispiel 123) werden mit 30 g Polyphosphorsäure 5 Stunden auf 100°C erhitzt. Nach dem Abkühlen nimmt man in Methylenchlorid/Natronlauge auf, trennt die organische Phase ab, dampft sie ein und chromatographiert den Rückstand über eine Kieselgelsäule (Petrolether-Essigester 7 : 3).

Man erhält 5,9 g (56 % d. Th.) Ethyl-1-(2-ethyl-6-vinyl-phenyl)-imidazol-5-carboxylat, ein farbloses Öl.

**$^1$H-NMR**

($CDCl_3$, 60 MHz) δ = 1,07 (t, J = 7 Hz, 3H, Phenyl-$CH_2\underline{CH_3}$), 1,20 (t, J = 7 Hz, 3H, O-$CH_2$-$\underline{CH_3}$), 2,27 (q, J = 7 Hz, 2H, Phenyl-$\underline{CH_2}$), 4,03 (q, J = 7 Hz, 2H, O-$CH_2$), 4,9 - 6,2 (m, 3H, -CH=$\underline{CH_2}$), 7,1 - 7,4 (m, 3H, Phenyl-$\underline{H}$), 7,5, 7,9 (2s, je 1H, Imidazol-$\underline{H}$) ppm.

**Beispiel 68**

**Ethyl-1-(2-butoxy-6-ethyl-phenyl)-imidazol-5-carboxylat**

13,5 g (0,07 mol) 2-Butoxy-6-ethylanilin werden mit 11,8 g (0,099 mol) Glyoxylsäureethylester bis zur Beendigung der Wasserbildung in 100 ml Toluol am Wasserabscheider erhitzt. Man dampft ein, trocknet im Hochvakuum, nimmt in 30 ml Dimethoxyethan auf und tropft die Lösung gemeinsam mit 13,4 g (0,069 mol) p-Toluol-sulfonylmethylisocyanid zu einer Suspension von 4,3 g (0,14 mol) 80 % Natriumhydrid (Weißöl) in 20 ml Dimethoxyethan bei -20°C zu. Nach Beendigung der Gasentwicklung wird durch Zutropfen von Wasser hydrolysiert, die Reaktionsprodukte mit Methylenchlorid extrahiert, und das Lösungsmittel eingedampft. Man nimmt in 50 ml Ethanol auf, gibt 20 g Kaliumcarbonat zu und rührt 2 Stunden bei 50°C. Nach dem Eindampfen wird in Toluol/Wasser aufgenommen, die organische Phase getrocknet, eingedampft und der Rückstand

über eine Kieselgelsäule chromatographiert (Petrolether-Essigester 8 : 2). Man erhält 12,1 g (55 % d. Th. bzgl. eingesetztem Anilin) Ethyl-1-(2-butoxy-6-ethyl-phenyl)-imidazol-5-carboxylat, ein hellgelbes Öl.

$^1$H-NMR

$CDCl_3$, 60 MHz) δ = 0,8 - 1,7 (sh, 13H), 2,33 (q, J = Hz, 2h, Phenyl-$\underline{CH_2}$), 3,83 (t, 2H, J = 6 Hz, -O-$\underline{CH_2}$-$CH_2$), 4,10 (q, J = 7 Hz, 2H, -O-$CH_2$-$CH_3$), 6,7 - 7,3 (m, 3H, Phenyl-$\underline{H}$) 7,43, 7,83 (2s, je 1 H, Imidazol-$\underline{H}$) ppm.

Weitere Beispiele sind in Tabelle 2 zusammengefaßt

Tabelle 2 Imidazolverbindungen

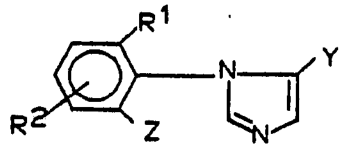

| Bsp. Nr. | $R^1$ | $R^2$ | Z | Y | Fp/Sdp. [°C] |
|---|---|---|---|---|---|
| 69 | Cl | H | Cl | $-COOH$ | 222-5 |
| 70 | " | " | " | $-COOCH_3$ | |
| 71 | " | " | " | $-COOC_2H_5$ | 123-4 |
| 72 | " | 4-Cl | " | $-COOH$ | |
| 73 | " | " | " | $-COOC_2H_5$ | 124-7 |
| 74 | " | H | $CH_3$ | $-COOH$ | 214-6 |
| 75 | " | " | " | $-COOC_2H_5$ | 135-40/ 0,1 Torr |
| 76 | " | " | " | $-CONH_2$ | |
| 77 | " | " | $C_2H_5$ | $-COOH$ | |
| 78 | " | " | " | $-COOC_2H_5$ | |
| 79 | " | " | " | $-CONH_2$ | |
| 80 | " | " | " | $-COOK$ | > 250 |
| 81 | Cl | 4-Cl | $CH_3$ | $-COOH$ | 208-15 |
| 82 | " | " | " | $-COOK$ | > 100 Zers. |
| 83 | " | " | " | $-COOCH_3$ | |
| 84 | " | " | " | $-COOC_2H_5$ | 65-70 |
| 85 | " | " | " | $-CONH_2$ | |
| 86 | " | " | " | $-CONHCH_3$ | |
| 87 | " | " | " | $-CON(CH_3)CH(CH_3)_2$ | |
| 88 | " | $4-CH_3$ | " | $-COOH$ | 199-200 |
| 89 | " | " | " | $-COOHN(-CH_2-CH_2OH)_3$ | |
| 90 | " | " | " | $-COOCH_3$ | 106-110 |
| 91 | " | " | " | $-COOC_2H_5$ | 100-103 |
| 92 | " | " | " | $-COO-n-C_3H_7$ | 85-7 |
| 93 | " | " | " | $-COO-i-C_3C_7$ | 99-103 |
| 94 | " | " | " | $-COO-CH_2-CF_3$ | |

| Bsp. Nr. | $R^1$ | $R^2$ | Z | Y | Fp/Sdp. [°C] |
|---|---|---|---|---|---|
| 95 | " | " | " | $-CONH_2$ | 210-3 |
| 96 | " | " | " | $-CONHCH_3$ | 188-190 |
| 97 | " | " | " | $-COON=C(CH_3)_2$ | |
| 98 | " | " | " | $-CONH-CH_2-CH(OCH_3)_2$ | Harz |
| 99 | " | " | " | -CON 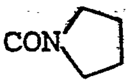 | |
| 100 | " | " | " | $-COSC_2H_5$ | |
| 101 | " | 5-Cl | $CH_3$ | -COOH | |
| 102 | " | " | " | $-COOC_2H_5$ | |
| 103 | " | " | " | $-CONH_2$ | |
| 104 | F | H | $CH_3$ | -COOH | |
| 105 | " | " | $C_2H_5$ | -COOH | |
| 106 | " | " | " | $-COOC_2H_5$ | |
| 107 | $OCH_3$ | " | $CH_3$ | $-COOC_2H_5$ | |
| 108 | " | " | " | $-CONH_2$ | |
| 109 | $OCH_3$ | " | $OCH_3$ | $-COOC_2H_5$ | |
| 110 | " | " | $CH_3$ | $-COOC_2H_5$ | |
| 111 | " | " | $C_2H_5$ | -COOH | |
| 112 | " | " | " | $-COOCH_3$ | |
| 113 | $O-n-C_4H_9$ | " | $CH_3$ | -COOH | |
| 114 | " | " | " | $-COOCH_3$ | |
| 115 | " | " | " | $-COOC_2H_5$ | |
| 116 | " | " | $C_2H_5$ | -COOH | |
| 117 | " | " | " | -COOK | |
| 118 | " | " | " | $-COOCH_3$ | |
| 119 | " | " | " | $-COON=C(CH_3)_2$ | |
| 120 | " | " | " | $-CONH_2$ | |
| 121 | " | " | " | $-CF_3$ | |
| 122 | $CH_2-OH$ | " | $C_2H_5$ | $-COOC_2H_5$ | 94-100 |
| 123 | $CH(OH)CH_3$ | " | $CH_3$ | -COOH | |
| 124 | " | " | " | $-COOC_2H_5$ | |
| 125 | $-CH=CH_2$ | " | $C_2H_5$ | -COOH | |

| Bsp. Nr. | $R^1$ | $R^2$ | Z | Y | Fp/Sdp. [°C] |
|---|---|---|---|---|---|
| 126 | " | " | " | $-COOCH_3$ | |
| 127 | " | " | " | $-CONH_2$ | |
| 128 | $-C(CH_3)=CH_2$ | " | $CH_3$ | $COOC_2H_5$ | |
| 129 | " | " | " | $-COOCH_3$ | |
| 130 | " | " | " | $-COOCH_2-CH_2-Cl$ | |
| 131 | " | " | " | $-CONH_2$ | |
| 132 | " | " | " | $-CF_3$ | |
| 133 | $-C(=O)CH_3$ | " | $C_2H_5$ | $-COOH$ | |
| 134 | " | " | " | $-COOCH_3$ | |
| 135 | $-C(CH_3)=CHBr$ | " | " | $-COOH$ | |
| 136 | " | " | " | $-COOCH_3$ | |
| 137 | " | " | " | $-CONH_2$ | |

**Biologische Beispiele**

**Wuchshemmung an Getreide**

In Schalenversuchen im Gewächshaus wurden junge Getreidepflanzen (Weizen, Gerste, Roggen) im 3-Blattstadium mit erfindungsgemäßen Verbindungen in verschiedenen Wirkstoffkonzentrationen (kg/ha) tropfnaß gespritzt.

Nachdem die unbehandelten Kontrollpflanzen eine Wuchshöhe von etwa 55 cm erreicht hatten, wurde bei allen Pflanzen der Zuwachs gemessen und die Wuchshemmung in % des Zuwachses der Kontrollpflanzen berechnet. Es wurde außerdem die phytotoxische Wirkung der Verbindungen beobachtet, wobei 100 % den Stillstand des Wuchstums und 0 % ein Wachstum entsprechend den unbehandelten Kontrollpflanzen bedeuten. Es zeigte sich, daß die Verbindungen sehr gute wachstumsregulierende Eigenschaften besitzen. Die Ergebnisse sind in der nachfolgenden Tabelle zusammengestellt.

**Tabelle 3**

| Verbindung nach Bsp.Nr. | Anwendungs-konz. kg/ha | Wuchshemmung (%) Weizen | Gerste | Roggen | Phytotox. Wirkung |
|---|---|---|---|---|---|
| 128 | 1,25 | 14 | 21 | 10 | keine |
| | 0,62 | 10 | 17 | 7 | Schäden |
| | 0,31 | 7 | 9 | 4 | |
| 81 | " | 22 | 34 | 14 | keine |
| | " | 17 | 26 | 12 | Schäden |
| | " | 10 | 15 | 9 | |
| 82 | " | 27 | 37 | 18 | keine |
| | " | 20 | 29 | 12 | Schäden |
| | " | 16 | 21 | 9 | |
| 116 | " | 16 | 19 | 14 | keine |
| | " | 12 | 15 | 10 | Schäden |
| | " | 9 | 11 | 7 | |
| 1 | " | 12 | 14 | 12 | keine |
| | " | 4 | 9 | 6 | Schäden |
| | " | 0 | 3 | 3 | |

**Wuchshemmung in Wasserreis**

Reispflanzen wurden in Töpfen im Gewächshaus bis zum 3-Blattstadium angezogen, und dann mit den erfindungsgemäßen Verbindungen behandelt. Die Substanzen wurden sowohl durch Spritzung appliziert als auch in das Wasser gegeben.
3 Wochen nach Behandlung wurde bei allen Pflanzen der Zuwachs gemessen und die Wuchshemmung in % des Zuwachses der Kontrollpflanzen berechnet. Es wurde außerdem auf eine mögliche phytotoxische Wirkung der Verbindungen geachtet.
Die Wuchshemmung wurde als prozentualer Wert ermittelt, wobei

100 % den Stillstand des Wachstums und 0 % ein Wachstum entsprechend dem der unbehandelten Kontrollpflanzen bedeuten. Die Ergebnisse sind in der nachfolgenden Tabelle zusammengefaßt.

**Tabelle 4**

| Verbindungen nach Bsp.Nr. | Anwendungs-konz. kg/ha | Wuchshemmung (%) | Phytotox. Wirkung |
|---|---|---|---|
| | 1,25 | 21 | keine |
| 75 | 0,62 | 13 | Schäden |
| | 0,31 | 7 | |
| | " | 20 | keine |
| 109 | " | 14 | Schäden |
| | " | 8 | |
| | " | 19 | keine |
| 63 | " | 15 | Schäden |
| | " | 9 | |
| | " | 22 | keine |
| 128 | " | 15 | Schäden |
| | " | 11 | |
| | " | 19 | keine |
| 81 | " | 15 | Schäden |
| | " | 9 | |
| | " | 18 | keine |
| 116 | " | 12 | Schäden |
| | " | 7 | |
| | " | 17 | keine |
| 68 | " | 12 | Schäden |
| | " | 9 | |
| | " | 14 | keine |
| 1 | " | 8 | Schäden |
| | " | 4 | |

**Patentansprüche:**

1. Verbindungen der Formel I

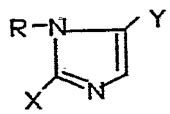

I

worin

R = einen Rest der Formel

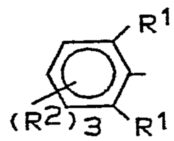

X = einen Rest der Formeln -SH,

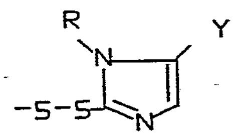

, $-S(O)_m-R^3$ oder Wasserstoff

Y = einen Rest der Formeln

$-CN$, $-\overset{O}{\overset{\|}{C}}-OR^4$, $-\overset{O}{\overset{\|}{C}}-SR^5$, $-\overset{S}{\overset{\|}{C}}-OR^5$, $-\overset{S}{\overset{\|}{C}}-SR^5$, $-\overset{O}{\overset{\|}{C}}-NR^6R^7$,

$-\overset{S}{\overset{\|}{C}}-N(R^6)_2$, $-\overset{NOR^6}{\overset{\|}{C}}-NH_2$, $-\overset{O}{\overset{\|}{C}}-R^6$, $-\overset{NOR^8}{\overset{\|}{C}}-R^6$, $-CH_2-OR^8$, $-CF_3$,

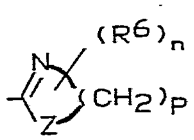 , 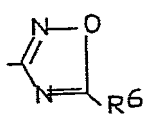 , 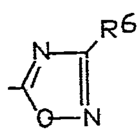 ,

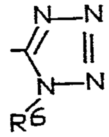 , 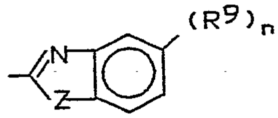

sowie die von $-\overset{O}{\overset{\|}{C}}-R^6$ abgeleiteten Bisulfitaddukte, Acetale, Ketale, Thioacetale oder Thioketale,

Z = O, S oder $N-R^6$

m = 0, 1 oder 2

n = 0, 1, 2, 3 oder 4

p = 2 oder 3

$R^1$= unabhängig voneinander $(C_1-C_4)$-Alkyl, das halogeniert sein kann, $(C_2-C_3)$-Alkenyl, $(C_2-C_3)$-Haloalkenyl, Acetyl, $(C_1-C_3)$-Hydroxyalkyl, $(C_1-C_4)$-Alkoxy oder Halogen,

$R^2$= unabhängig voneinander Wasserstoff, $(C_1-C_4)$-Alkyl, $(C_1-C_4)$-Alkoxy oder Halogen,

$R^3$= $(C_1-C_4)$-Alkyl, $(C_2-C_4)$-Alkenyl, $(C_1-C_4)$-Alkoxycarbonyl-$(C_1-C_2)$alkyl, Phenyl oder Benzyl,

$R^4$= Wasserstoff, $(C_1-C_{12})$-Alkyl, $(C_1-C_{12})$-Alkyl, das ein- bis dreifach durch Hydroxy, Halogen, $(C_1-C_6)$-Alkoxy, $(C_1-C_4)$-Alkoxy-$(C_1-C_4)$alkoxy, $(C_1-C_4)$-Alkylthio, $(C_1-C_4)$-Alkylsulfinyl, $(C_1-C_4)$-Alkylsulfonyl, Mono- oder Di-$(C_1-C_4$-alkyl)amino, Cyano, Aminocarbonyl, $(C_1-C_4)$-Alkanoyl, $(C_1-C_4$-Alkoxy)carbonyl, Cyclo-$(C_3-C_7)$alkyl, Tri$(C_1-C_4$-alkyl)silyl, Benzyloxy, Benzyloxyethoxy, Phenyl oder Phenyl, das durch Halogen oder $(C_1-C_4)$-Alkyl ein- oder mehrfach substituiert ist, durch Phenoxy, Phenylthio, die beide durch Halogen oder

$(C_1-C_4)$-Alkyl ein- oder mehrfach substituiert sein können, durch Oxiranyl, Tetrahydrofuryl, Triazolyl, Pyridinyl, Imidazolyl, Carboxy, Carboxylat mit einem für die Landwirtschaft einsetzbaren Kation oder durch den Rest $-O-N=C(CH_3)_2$ substituiert ist, $(C_3-C_6)$-Alkenyl, halogeniertes $(C_3-C_6)$-Alkenyl, unsubstituiertes oder durch Halogen oder $(C_1-C_4)$-Alkyl substituiertes Cyclo-$(C_3-C_7)$alkyl, unsubstituiertes oder durch Halogen oder $(C_1-C_4)$-Alkyl substituiertes Cyclo-$(C_5-C_7)$alkenyl, $(C_3-C_6)$-Alkinyl, 1,2-Epoxy-prop-3-yl,

Phenyl oder Phenyl, das ein- oder zweifach durch Halogen, Nitro, Cyano, $(C_1-C_4)$-Alkyl, $(C_1-C_4$-Alkoxy$)$-carbonyl oder $(C_1-C_4)$-Alkoxy substituiert ist, $(C_1-C_4$-Alkyl$)$carbonyl, Phenylcarbonyl, wobei der Phenylring durch Halogen, Nitro, Cyano oder $(C_1-C_4)$-Alkyl substituiert sein kann,

einen Rest der Formeln $-N=C(R^{10})_2$, $-NR^6R^{11}$,

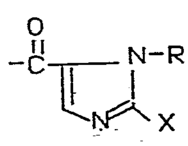 , 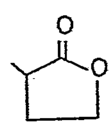 , 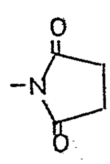 , 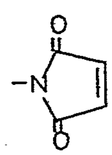 , 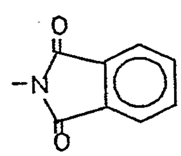

oder ein für die Landwirtschaft einsetzbares Kation,

$R^5$= H, $(C_1-C_{12})$-Alkyl oder $(C_1-C_{12})$-Alkyl, das bis zu zweifach durch $(C_1-C_4)$-Alkoxy-ethoxy, Cyclo-$(C_3-C_6)$-alkyl, Benzyloxy, Phenyl, Phenoxy, $(C_1-C_4)$-Alkylthio, $(C_1-C_4$-Alkoxy$)$carbonyl, Carboxy oder Carboxylat mit einem für die Landwirtschaft einsetzbaren Kation, substituiert ist, Phenyl oder im Falle $-CS-OR^5$ ein für die Landwirtschaft einsetzbares Kation,

$R^6$= jeweils unabhängig voneinander Wasserstoff, $(C_1-C_6)$-Alkyl, Phenyl, Benzyl oder Methylphenyl,

$R^7$= Wasserstoff, ($C_1$-$C_{12}$)-Alkyl oder ($C_1$-$C_{12}$)-Alkyl, das bis zu zweifach, durch ($C_1$-$C_6$)-Alkoxy, ($C_1$-$C_4$)-Alkoxyethoxy, Hydroxy, Halogen, Cyclo-($C_3$-$C_6$)alkyl, Benzyloxy, Cyano, Aminocarbonyl, Carboxy, ($C_1$-$C_4$-Alkoxy)carbonyl, Formyl, Phenyl oder Phenoxy substituiert ist,
Phenyl oder Phenyl, das bis zu zweifach durch Halogen, Nitro, Cyano, ($C_1$-$C_4$)-Alkyl oder ($C_1$-$C_4$)-Alkoxy substituiert ist,
($C_3$-$C_6$)-Alkenyl, ($C_3$-$C_6$)-Cycloalkyl, einen Rest der Formeln $-NR^6R^{12}$, $-OR^6$, $-NH-CONH_2$, $-NH-CS-NH_2$ oder $-SO_2R^6$ oder

$R^6$ und $R^7$ gemeinsam mit dem Stickstoffatom an das sie gebunden sind, einen gesättigten oder ungesättigten gegebenenfalls benzokondensierten drei- bis siebengliedrigen Ring, der bis zu drei Heteroatome aus der Gruppe O, N und S enthält und der unsubstituiert oder durch ($C_1$-$C_4$)-Alkyl oder Halogen substituiert ist und eine Carbonylgruppe enthalten kann,

$R^8$= jeweils unabhängig voneinander H, unsubstituiertes oder durch Phenyl, Halogenphenyl, Nitrophenyl, Cyanophenyl, ($C_1$-$C_4$)-Alkylphenyl oder ($C_1$-$C_4$)-Alkoxyphenyl, Hydroxy, Cyano, ($C_1$-$C_4$-Alkoxy)-carbonyl, ($C_1$-$C_4$)-Alkylthio, ($C_1$-$C_4$)-Alkoxy, Cyclo-($C_5$-$C_7$)alkyl oder Benzyloxy substituiertes ($C_1$-$C_{12}$)-Alkyl, Cyclo-($C_5$-$C_8$)alkyl, ($C_3$-$C_6$)-Alkenyl, Halogen-($C_3$-$C_6$)alkenyl, ($C_3$-$C_6$)-Alkinyl, Cyclo-($C_5$-$C_6$)alkenyl, ($C_1$-$C_6$-Alkyl)-carbonyl, Halogen-($C_1$-$C_6$-alkyl)carbonyl mit 1 bis 3 Halogenatomen, ($C_1$-$C_6$-Alkylamino)carbonyl, Benzoyl, Halogenbenzoyl oder Methylbenzoyl,

$R^9$= jeweils unabhängig voneinander H, Halogen, ($C_1$-$C_4$)-Alkyl, Nitro oder Cyano,

$R^{10}$= unabhängig voneinander $(C_1-C_6)$-Alkyl, Cyclo-$(C_3-C_6)$-alkyl, $(C_3-C_6)$-Alkenyl, Phenyl, Benzyl oder beide Reste $R^{10}$ gemeinsam mit dem Kohlenstoff, an das sie gebunden sind, ein substituiertes oder durch Methyl oder Halogen substituiertes Cyclo-$(C_5-C_7)$alkyl,

$R^{11}$= $(C_1-C_4)$-Alkyl, Phenyl, $(C_1-C_6$-Alkyl)carbonyl, Benzyl, Benzoyl, Halogenbenzyl, Halogenbenzoyl oder Methylbenzoyl und

$R^{12}$= H, $(C_1-C_4)$-Alkyl, Formyl, $(C_1-C_6$-Alkyl)carbonyl, Benzoyl, Halogenbenzoyl, Methylbenzoyl oder Trihalogenacetyl, bedeuten

sowie deren für die landwirtschaftlichen Zwecke verträglichen Salze und Quaternisierungsprodukte, mit der Maßgabe, daß nur ein Rest $R^1$ $(C_1-C_4)$-Alkyl sein darf.

2. Verbindungen der Formel I

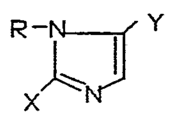

I,

worin

R = einen Rest der Formel

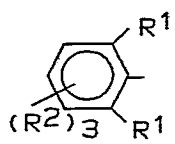

X = einen Rest der Formeln -SH,

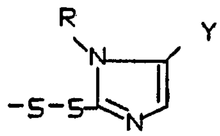 oder Wasserstoff

Y = einen Rest der Formeln

$-CN$, $-\overset{O}{\overset{\|}{C}}-OR^4$, $-\overset{O}{\overset{\|}{C}}-SR^5$, $-\overset{O}{\overset{\|}{C}}-NR^6R^7$, $-\overset{O}{\overset{\|}{C}}-R^6$, $-CF_3$,

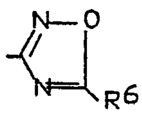 , 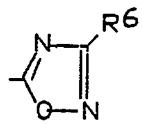 , 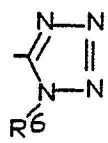 ,

$R^1$= unabhängig voneinander ($C_1$-$C_3$)-Alkyl, das halogeniert sein kann, ($C_2$-$C_3$)-Alkenyl, ($C_2$-$C_3$)-Haloalkenyl, Acetyl, ($C_1$-$C_3$)-Hydroxyalkyl, ($C_1$-$C_4$)-Alkoxy oder Halogen,

$R^2$= unabhängig voneinander Wasserstoff, ($C_1$-$C_4$)-Alkyl oder Halogen,

$R^4$= Wasserstoff, ($C_1$-$C_6$)-Alkyl, ($C_1$-$C_6$)-Alkyl, das ein- bis dreifach durch Hydroxy, Halogen, ($C_1$-$C_6$)-Alkoxy, ($C_1$-$C_4$)-Alkylthio, Cyano, Trimethylsilyl, Benzyloxy oder Phenyl substituiert ist, ($C_3$-$C_6$)-Alkenyl, halogeniertes ($C_3$-$C_6$)-Alkenyl, Cyclo-($C_3$-$C_7$)alkyl, ($C_3$-$C_6$)-Alkinyl, Phenyl oder einen Rest der Formeln $-N{=}C(R^{10})_2$,

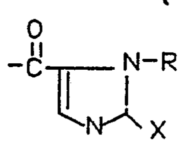 ,

oder ein für die Landwirtschaft einsetzbares Kation,

$R^5$= H, $(C_1-C_6)$-Alkyl,

$R^6$= Wasserstoff, $(C_1-C_6)$-Alkyl,

$R^7$= Wasserstoff, $(C_1-C_6)$-Alkyl oder $(C_1-C_6)$-Alkyl, das bis zu zweifach, durch $(C_1-C_2)$-Alkoxy substituiert ist, Phenyl, $(C_3-C_6)$-Alkenyl, $(C_3-C_6)$-Cycloalkyl, einen Rest der Formeln $-NR^6R^{12}$, $-OR^6$,

$R^{10}$= unabhängig voneinander $(C_1-C_4)$-Alkyl,

$R^{12}$= H, $(C_1-C_4)$-Alkyl, bedeuten

sowie deren für die landwirtschaftlichen Zwecke verträglichen Salze und Quaternisierungsprodukte, mit der Maßgabe, daß nur ein Rest $R^1$ $(C_1-C_4)$-Alkyl sein darf.

3. Verbindungen der Formel I

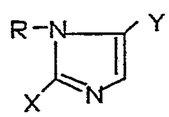

I

worin

R = einen Rest der Formel

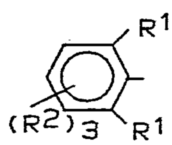

X = Wasserstoff

Y = -C-OR$^4$, -C-NR$^6$R$^7$ oder -C-H
O O O

$R^1$= unabhängig voneinander $(C_1-C_3)$-Alkyl, Trifluormethyl, Vinyl, $(C_1-C_4)$-Alkoxy, Fluor oder Chlor,

$R^2$= unabhängig voneinander Wasserstoff, Methyl oder Chlor,

$R^4$= Wasserstoff, $(C_1-C_6)$-Alkyl, $(C_1-C_6)$-Alkyl, das ein- bis dreifach durch Halogen oder einfach durch Trimethylsilyl substituiert ist, Allyl, Propargyl, einen Rest der Formeln $-N=C(R^{10})_2$,

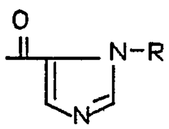

oder ein für die Landwirtschaft einsetzbares Kation,

$R^6$= Wasserstoff, $(C_1-C_6)$-Alkyl,

$R^7$= Wasserstoff, $(C_1-C_6)$-Alkyl oder $(C_1-C_6)$-Alkyl, das bis zu zweifach durch Methoxy, Ethoxy substituiert ist, Allyl, $(C_3-C_6)$-Cycloalkyl, einen Rest der Formeln $-NR^6R^{12}$ oder $-OR^6$,

$R^{10}$= $(C_1-C_4)$-Alkyl und

$R^{12}$= Wasserstoff oder Methyl, bedeuten

sowie deren für die landwirtschaftlichen Zwecke verträglichen Salze und Quaternisierungsprodukte, mit der Maßgabe, daß nur ein Rest $R^1$ $(C_1-C_3)$-Alkyl sein darf.

4. Verfahren zur Herstellung der Verbindungen der allgemeinen Formel I gemäß einem der Ansprüche 1 - 3, dadurch gekennzeichnet, daß man

a) einen Bisformylester der Formeln IIa oder IIb

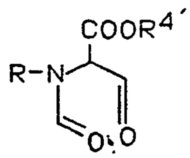

IIa

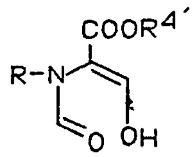

IIb

wobei $R^{4'}$ = $(C_1-C_{12})$-Alkyl bedeutet,
($a_1$) mit einem $(C_1-C_3)$-Carbonsäureamid cyclisiert oder
($a_2$) mit einem Alkali- oder Ammoniumrhodanid zu einem 2-Mercaptoimidazol-Derivat umsetzt und dieses gegebenenfalls mit Salpetersäure und Natriumnitrit entschwefelt
und die erhaltenen Verbindungen derivatisiert;

b) eine Imidazolverbindung der Formel III,

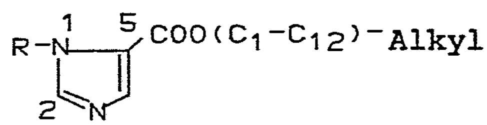

III

mit einer starken Base in 2-Stellung des Imidazolrings metalliert und anschließend mit einem Disulfid der Formel $R^3$-S-S-$R^3$ zu einer Verbindung der Formel I mit X = -S-$R^3$ und Y = COO($C_1$-$C_{12}$-alkyl) umsetzt, und die erhaltenen Verbindungen derivatisiert.

c) Eine Imidazolverbindung der Formel IV

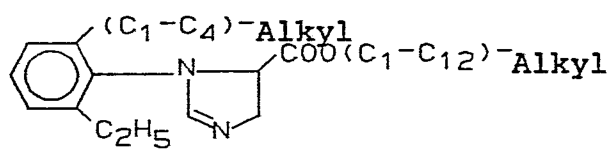

IV

an der Ethylgruppe oxidiert und so eine Verbindung der Formel I mit $R^1$ = Acetyl erhält und die erhaltenen Verbindungen ggf. derivatisiert oder

d) eine Iminoverbindung der Formeln Va oder Vb

R-N=CH-COO$(C_1-C_4)$-Alkyl Va

R-N=CH-$CF_3$ Vb

mit p-Toluolsulfonylmethylisocyanid umsetzt und die erhaltenen Verbindungen der Formel I gegebenenfalls derivatisiert.

5. Pflanzenwachstumsregulierende Mittel mit einem wirksamen Gehalt an einer Verbindung der allgemeinen Formel (I) gemäß einem der Ansprüche 1 - 3.

6. Verwendung von Verbindungen der allgemeinen Formel (I) gemäß einem der Ansprüche 1 - 3 als Wachstumsregulatoren für Pflanzen.

7. Verfahren zur Wachstumsregulierung von Pflanzen, dadurch gekennzeichnet, daß man auf die Pflanzen oder die Anbaufläche eine wirksame Menge einer Verbindung der allgemeinen Formel (I) gemäß einem der Ansprüche 1 - 3 appliziert.

Patentansprüche Griechenland, Österreich und Spanien:

1. Verfahren zur Herstellung von Verbindungen der Formel I

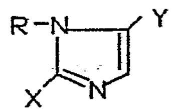

I,

worin

R = einen Rest der Formel

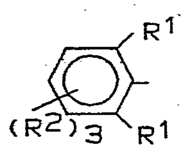

X = einen Rest der Formeln -SH,

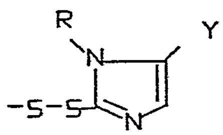

, $-S(O)_m-R^3$ oder Wasserstoff

Y = einen Rest der Formeln

$-CN$, $-\overset{O}{\overset{\|}{C}}-OR^4$, $-\overset{O}{\overset{\|}{C}}-SR^5$, $-\overset{S}{\overset{\|}{C}}-OR^5$, $-\overset{S}{\overset{\|}{C}}-SR^5$, $-\overset{O}{\overset{\|}{C}}-NR^6R^7$,

$-\overset{S}{\overset{\|}{C}}-N(R^6)_2$, $-\overset{NOR^6}{\overset{\|}{C}}-NH_2$, $-\overset{O}{\overset{\|}{C}}-R^6$, $-\overset{NOR^8}{\overset{\|}{C}}-R^6$, $-CH_2-OR^8$, $-CF_3$,

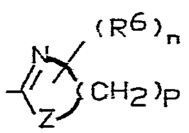 , 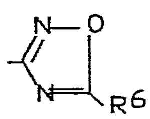 , 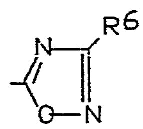 ,

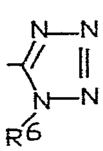 , 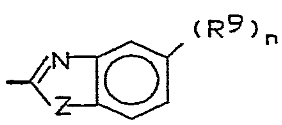

sowie die von $-\overset{O}{\overset{\|}{C}}-R^6$ abgeleiteten Bisulfitaddukte, Acetale, Ketale, Thiocetale oder Thioketale,

Z = O, S oder $N-R^6$

m = 0, 1 oder 2

n = 0, 1, 2, 3 oder 4

p = 2 oder 3

$R^1$= unabhängig voneinander $(C_1-C_4)$-Alkyl, das halogeniert sein kann, $(C_2-C_3)$-Alkenyl, $(C_2-C_3)$-Haloalkenyl, Acetyl, $(C_1-C_3)$-Hydroxyalkyl, $(C_1-C_4)$-Alkoxy oder Halogen,

$R^2$= unabhängig voneinander Wasserstoff, $(C_1-C_4)$-Alkyl, $(C_1-C_4)$-Alkoxy oder Halogen,

$R^3$= $(C_1-C_4)$-Alkyl, $(C_2-C_4)$-Alkenyl, $(C_1-C_4)$-Alkoxycarbonyl-$(C_1-C_2)$alkyl, Phenyl oder Benzyl,

$R^4$= Wasserstoff, $(C_1-C_{12})$-Alkyl, $(C_1-C_{12})$-Alkyl, das ein- bis dreifach durch Hydroxy, Halogen, $(C_1-C_6)$-Alkoxy, $(C_1-C_4)$-Alkoxy-$(C_1-C_4)$alkoxy, $(C_1-C_4)$-Alkylthio, $(C_1-C_4)$-Alkylsulfinyl, $(C_1-C_4)$-Alkylsulfonyl, Mono- oder Di-$(C_1-C_4$-alkyl)amino, Cyano, Aminocarbonyl, $(C_1-C_4)$-Alkanoyl, $(C_1-C_4$-Alkoxy)carbonyl, Cyclo-$(C_3-C_7)$alkyl, Tri$(C_1-C_4$-alkyl)silyl, Benzyloxy, Benzyloxyethoxy, Phenyl oder Phenyl, das durch Halogen oder $(C_1-C_4)$-Alkyl ein- oder mehrfach substituiert ist, durch Phenoxy, Phenylthio, die beide durch Halogen oder

$(C_1-C_4)$-Alkyl ein- oder mehrfach substituiert sein können, durch Oxiranyl, Tetrahydrofuryl, Triazolyl, Pyridinyl, Imidazolyl, Carboxy, Carboxylat mit einem für die Landwirtschaft einsetzbaren Kation oder durch den Rest $-O-N=C(CH_3)_2$ substituiert ist, $(C_3-C_6)$-Alkenyl, halogeniertes $(C_3-C_6)$-Alkenyl, unsubstituiertes oder durch Halogen oder $(C_1-C_4)$-Alkyl substituiertes Cyclo-$(C_3-C_7)$alkyl, unsubstituiertes oder durch Halogen oder $(C_1-C_4)$-Alkyl substituiertes Cyclo-$(C_5-C_7)$alkenyl, $(C_3-C_6)$-Alkinyl, 1,2-Epoxy-prop-3-yl,

Phenyl oder Phenyl, das ein- oder zweifach durch Halogen, Nitro, Cyano, $(C_1-C_4)$-Alkyl, $(C_1-C_4$-Alkoxy)-carbonyl oder $(C_1-C_4)$-Alkoxy substituiert ist, $(C_1-C_4$-Alkyl)carbonyl, Phenylcarbonyl, wobei der Phenylring durch Halogen, Nitro, Cyano oder $(C_1-C_4)$-Alkyl substituiert sein kann,

einen Rest der Formeln $-N=C(R^{10})_2$, $-NR^6R^{11}$,

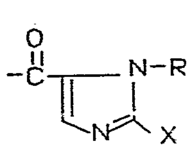 , 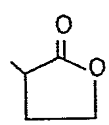 , 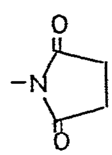 , 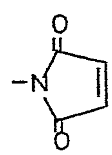 , 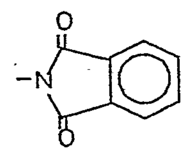

oder ein für die Landwirtschaft einsetzbares Kation,

$R^5$= H, $(C_1-C_{12})$-Alkyl oder $(C_1-C_{12})$-Alkyl, das bis zu zweifach durch $(C_1-C_4)$-Alkoxy-ethoxy, Cyclo-$(C_3-C_6)$-alkyl, Benzyloxy, Phenyl, Phenoxy, $(C_1-C_4)$-Alkylthio, $(C_1-C_4$-Alkoxy)carbonyl, Carboxy oder Carboxylat mit einem für die Landwirtschaft einsetzbaren Kation, substituiert ist, Phenyl oder im Falle $-CS-OR^5$ ein für die Landwirtschaft einsetzbares Kation,

$R^6$= jeweils unabhängig voneinander Wasserstoff, $(C_1-C_6)$-Alkyl, Phenyl, Benzyl oder Methylphenyl,

$R^7$= Wasserstoff, ($C_1$-$C_{12}$)-Alkyl oder ($C_1$-$C_{12}$)-Alkyl, das bis zu zweifach, durch ($C_1$-$C_6$)-Alkoxy, ($C_1$-$C_4$)-Alkoxyethoxy, Hydroxy, Halogen, Cyclo-($C_3$-$C_6$)alkyl, Benzyloxy, Cyano, Aminocarbonyl, Carboxy, ($C_1$-$C_4$-Alkoxy)carbonyl, Formyl, Phenyl oder Phenoxy substituiert ist,
Phenyl oder Phenyl, das bis zu zweifach durch Halogen, Nitro, Cyano, ($C_1$-$C_4$)-Alkyl oder ($C_1$-$C_4$)-Alkoxy substituiert ist,
($C_3$-$C_6$)-Alkenyl, ($C_3$-$C_6$)-Cycloalkyl, einen Rest der Formeln $-NR^6R^{12}$, $-OR^6$, $-NH-CONH_2$, $-NH-CS-NH_2$ oder $-SO_2R^6$ oder

$R^6$ und $R^7$ gemeinsam mit einem Stickstoffatom an das sie gebunden sind, einen gesättigten oder ungesättigten gegebenenfalls benzokondensierten drei- bis siebengliedrigen Ring, der bis zu drei Heteroatome aus der Gruppe O, N und S enthält und der unsubstituiert oder durch ($C_1$-$C_4$)-Alkyl oder Halogen substituiert ist und eine Carbonylgruppe enthalten kann,

$R^8$= jeweils unabhängig voneinander H, unsubstituiertes oder durch Phenyl, Halogenphenyl, Nitrophenyl, Cyanophenyl, ($C_1$-$C_4$)-Alkylphenyl oder ($C_1$-$C_4$)-Alkoxyphenyl, Hydroxy, Cyano, ($C_1$-$C_4$-Alkoxy)-carbonyl, ($C_1$-$C_4$)-Alkylthio, ($C_1$-$C_4$)-Alkoxy, Cyclo-($C_5$-$C_7$)alkyl oder Benzyloxy substituiertes ($C_1$-$C_{12}$)-Alkyl, Cyclo-($C_5$-$C_8$)alkyl, ($C_3$-$C_6$)-Alkenyl, Halogen-($C_3$-$C_6$)alkenyl, ($C_3$-$C_6$)-Alkinyl, Cyclo-($C_5$-$C_6$)alkenyl, ($C_1$-$C_6$-Alkyl)-carbonyl, Halogen-($C_1$-$C_6$-alkyl)carbonyl mit 1 bis 3 Halogenatomen, ($C_1$-$C_6$-Alkylamino)carbonyl, Benzoyl, Halogenbenzoyl oder Methylbenzoyl,

$R^9$= jeweils unabhängig voneinander H, Halogen, ($C_1$-$C_4$)-Alkyl, Nitro oder Cyano,

$R^{10}$= unabhängig voneinander $(C_1-C_6)$-Alkyl, Cyclo-$(C_3-C_6)$-alkyl, $(C_3-C_6)$-Alkenyl, Phenyl, Benzyl oder beide Reste $R^{10}$ gemeinsam mit dem Kohlenstoff, an das sie gebunden sind, ein substituiertes oder durch Methyl oder Halogen substituiertes Cyclo-$(C_5-C_7)$alkyl,

$R^{11}$= $(C_1-C_4)$-Alkyl, Phenyl, $(C_1-C_6$-Alkyl)carbonyl, Benzyl, Benzoyl, Halogenbenzyl, Halogenbenzoyl oder Methylbenzoyl und

$R^{12}$= H, $(C_1-C_4)$-Alkyl, Formyl, $(C_1-C_6$-Alkyl)carbonyl, Benzoyl, Halogenbenzoyl, Methylbenzoyl oder Trihalogenacetyl, bedeuten

sowie deren für die landwirtschaftlichen Zwecke verträglichen Salze und Quaternisierungsprodukte, mit der Maßgabe, daß nur ein Rest $R^1$ $(C_1-C_4)$-Alkyl sein darf, dadurch gekennzeichnet, daß man

a) einen Bisformylester der Formeln IIa oder IIb

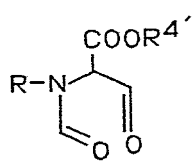
IIa

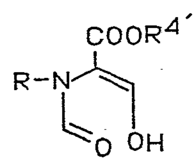
IIb

wobei $R^{4'}$= $(C_1-C_{12})$-Alkyl bedeutet,

($a_1$) mit einem $(C_1-C_3)$-Carbonsäureamid cyclisiert oder

($a_2$) mit einem Alkali- oder Ammoniumrhodanid zu einem 2-Mercaptoimidazol-Derivat umsetzt und dieses gegebenenfalls mit Salpetersäure und Natriumnitrit entschwefelt

und die erhaltenen Verbindungen derivatisiert;

b) eine Imidazolverbindung der Formel III,

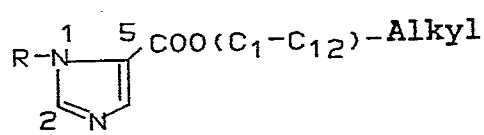
III

mit einer starken Base in 2-Stellung des Imidazolrings metalliert und anschließend mit einem Disulfid der Formel $R^3$-S-S-$R^3$ zu einer Verbindung der Formel I mit X = -S-$R^3$ und Y = COO($C_1$-$C_{12}$-alkyl) umsetzt, und die erhaltenen Verbindungen derivatisiert;

c) eine Imidazolverbindung der Formel IV

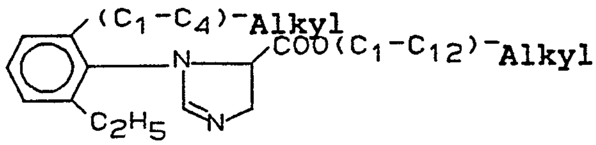

IV

an der Ethylgruppe oxidiert und so eine Verbindung der Formel I mit $R^1$ = Acetyl erhält und die erhaltenen Verbindungen ggf. derivatisiert,

d) eine Iminoverbindung der Formeln Va oder Vb

R-N=CH-COO($C_1$-$C_4$)-Alkyl Va

R-N=CH-$CF_3$ Vb

mit p-Toluolsulfonylmethylisocyanid umsetzt und die erhaltenen Verbindungen der Formel I gegebenenfalls derivatisiert.

2. Verfahren gemäß Anspruch 1, dadurch gekennzeichnet, daß in Formel I von Anspruch 1

R = einen Rest der Formel

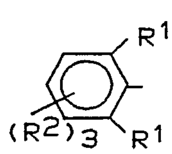

X = einen Rest der Formeln -SH,

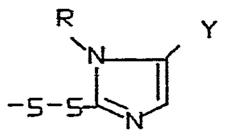 oder Wasserstoff

Y = einen Rest der Formeln

$-CN$, $-\overset{O}{\overset{\|}{C}}-OR^4$, $-\overset{O}{\overset{\|}{C}}-SR^5$, $-\overset{O}{\overset{\|}{C}}-NR^6R^7$, $-\overset{O}{\overset{\|}{C}}-R^6$, $-CF_3$,

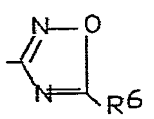 , 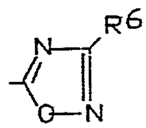 , 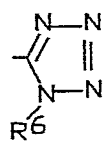 ,

$R^1$= unabhängig voneinander $(C_1-C_3)$-Alkyl, das halogeniert sein kann, $(C_2-C_3)$-Alkenyl, $(C_2-C_3)$-Haloalkenyl, Acetyl, $(C_1-C_3)$-Hydroxyalkyl, $(C_1-C_4)$-Alkoxy oder Halogen,

$R^2$= unabhängig voneinander Wasserstoff, $(C_1-C_4)$-Alkyl oder Halogen,

$R^4$= Wasserstoff, $(C_1-C_6)$-Alkyl, $(C_1-C_6)$-Alkyl, das ein- bis dreifach durch Hydroxy, Halogen, $(C_1-C_6)$-Alkoxy, $(C_1-C_4)$-Alkylthio, Cyano, Trimethylsilyl, Benzyloxy oder Phenyl substituiert ist,
$(C_3-C_6)$-Alkenyl, halogeniertes $(C_3-C_6)$-Alkenyl, Cyclo-$(C_3-C_7)$alkyl, $(C_3-C_6)$-Alkinyl, Phenyl oder einen Rest der Formeln $-N=C(R^{10})_2$,

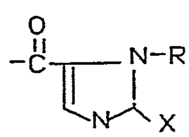 ,

oder ein für die Landwirtschaft einsetzbares Kation,

$R^5$= H, ($C_1$-$C_6$)-Alkyl,

$R^6$= Wasserstoff, ($C_1$-$C_6$)-Alkyl,

$R^7$= Wasserstoff, ($C_1$-$C_6$)-Alkyl oder ($C_1$-$C_6$)-Alkyl, das bis zu zweifach, durch ($C_1$-$C_2$)-Alkoxy substituiert ist, Phenyl, ($C_3$-$C_6$)-Alkenyl, ($C_3$-$C_6$)-Cycloalkyl, einen Rest der Formeln $-NR^6R^{12}$, $-OR^6$,

$R^{10}$= unabhängig voneinander ($C_1$-$C_4$)-Alkyl,

$R^{12}$= H, ($C_1$-$C_4$)-Alkyl, bedeuten

sowie deren für die landwirtschaftlichen Zwecke verträglichen Salze und Quaternisierungsprodukte, mit der Maßgabe, daß nur ein Rest $R^1$ ($C_1$-$C_4$)-Alkyl sein darf.

3. Verfahren gemäß Anspruch 1, dadurch gekennzeichnet, daß in Formel I von Anspruch 1

R = einen Rest der Formel

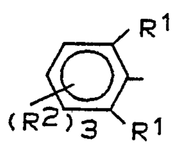

X = Wasserstoff

Y = $-\underset{\overset{\|}{O}}{C}-OR^4$, $-\underset{\overset{\|}{O}}{C}-NR^6R^7$ oder $-\underset{\overset{\|}{O}}{C}-H$

$R^1$= unabhängig voneinander ($C_1$-$C_3$)-Alkyl, Trifluormethyl, Vinyl, ($C_1$-$C_4$)-Alkoxy, Fluor oder Chlor,

$R^2$= unabhängig voneinander Wasserstoff, Methyl oder Chlor,

$R^4$= Wasserstoff, $(C_1-C_6)$-Alkyl, $(C_1-C_6)$-Alkyl, das ein- bis dreifach durch Halogen oder einfach durch Trimethylsilyl substituiert ist, Allyl, Propargyl, einen Rest der Formeln $-N=C(R^{10})_2$,

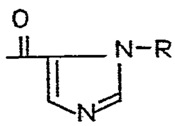

oder ein für die Landwirtschaft einsetzbares Kation,

$R^6$= Wasserstoff, $(C_1-C_6)$-Alkyl,

$R^7$= Wasserstoff, $(C_1-C_6)$-Alkyl oder $(C_1-C_6)$-Alkyl, das bis zu zweifach durch Methoxy, Ethoxy substituiert ist, Allyl, $(C_3-C_6)$-Cycloalkyl, einen Rest der Formeln $-NR^6R^{12}$ oder $-OR^6$,

$R^{10}$= $(C_1-C_4)$-Alkyl und

$R^{12}$= Wasserstoff oder Methyl, bedeuten

sowie deren für die landwirtschaftlichen Zwecke verträglichen Salze und Quaternisierungsprodukte, mit der Maßgabe, daß nur ein Rest $R^1$ $(C_1-C_3)$-Alkyl sein darf.

4. Pflanzenwachstumsregulierende Mittel mit einem wirksamen Gehalt an einer Verbindung der allgemeinen Formel (I) gemäß einem der Ansprüche 1 - 3.

5. Verwendung von Verbindungen der allgemeinen Formel (I) gemäß einem der Ansprüche 1 - 3 als Wachstumsregulatoren für Pflanzen.

6. Verfahren zur Wachstumsregulierung von Pflanzen, dadurch gekennzeichnet, daß man auf die Pflanzen oder die Anbaufläche eine wirksame Menge einer Verbindung der allgemeinen Formel (I) gemäß einem der Ansprüche 1 - 3 appliziert.